# EUROPEAN PATENT SPECIFICATION

(11) **EP 3 236 953 B1**
(45) Date of publication and mention of the grant of the patent: **11.11.2020**
(21) Application number: 15826209.7
(22) Date of filing: 23.12.2015
(51) Int. Cl.: A61K 9/50, A61K 9/28, A61K 31/506, A61K 31/519, A61K 9/48

(54) **SITE SPECIFIC DOSING OF A BTK INHIBITOR**
ORTSSPEZIFISCHE DOSIERUNG EINES BTK-INHIBITOR
DOSAGE SPÉCIFIQUE DE SITE D'UN INHIBITEUR DE BTK

(30) Priority: 24.12.2014 US 201462096809 P
(43) Date of publication of application: 01.11.2017
(73) Proprietor: Principia Biopharma Inc., S. San Francisco, CA 94080 (US)
(72) Inventor: NUNN, Philipp, Mountain View, CA 94014 (US); BERNER, Bret, Seattle, WA 98116 (US); MASJEDIZADEH, Mohammad, San Jose, CA 95124 (US)
(74) Representative: J A Kemp LLP
(86) International application number: PCT/US2015/000303
(87) International publication number: WO 2016/105531

(56) References cited:
- WO-A1-2013/191965
- WO-A1-2014/039899
- WILDING I R ET AL: "Targeting of drugs and vaccines to the gut", PHARMACOLOGY AND THERAPEUTICS, ELSEVIER, GB, vol. 62, no. 1-2, 1 January 1994 (1994-01-01), pages 97-124, XP025557223, ISSN: 0163-7258, DOI: 10.1016/0163-7258(94)90006-X [retrieved on 1994-01-01]
- SANTUS G ET AL: "Osmotic drug delivery: a review of the patent literature", JOURNAL OF CONTROLLED RELEASE, ELSEVIER, AMSTERDAM, NL, vol. 35, no. 1, 1 July 1995 (1995-07-01), pages 1-21, XP004037488, ISSN: 0168-3659, DOI: 10.1016/0168-3659(95)00013-X

## Description

### Field

Disclosed herein are formulations and methods of site specific administration of Compound (I) or a pharmaceutically acceptable salt thereof. Compound (I) is a potent BTK inhibitor and hence can be useful for the treatment of diseases such as cancer, autoimmune, and inflammatory diseases.

### Background

Therapeutic agents can be administered to patients via several different routes such as oral, topical, intravenous, subcutaneous, inhalation, etc. Oral dosing of the therapeutics is by far the most preferred route of administration and offers multiple advantages over other routes of administration. Orally delivered drugs are easily self-administered thereby resulting in increased patient compliance and obviating the requirement for specialized delivery devices for injectable or inhaled therapies or delivery in a therapeutic setting. It is typically the safest route of getting a drug into the body since it does not require complicated devices or puncturing of body surfaces or membranes. Additionally dosage is readily controlled, which can be challenging for other modes of administration such as inhaled therapies.

Despite numerous advantages, obtaining consistent and adequate circulating levels of drug with oral dosing can be challenging due to, among other things such as poor aqueous solubility, slow dissolution rate in biological fluids, poor stability of drug at physiological pH, poor permeation through biomembrane, extensive presystemic metabolism, inadequate or inconsistent systemic absorption between individuals or within specific regions of the gastrointestinal system. Additionally drug absorption can vary from therapy to therapy and depend upon numerous factors such as whether the patient is in a fed or fasted state at the time of administration, or whether the drug is taken concurrently with other medications. From a safety standpoint, minimizing the total dosage requirement for efficacy as well as reducing variability in absorption should allow for fewer unwanted side effects. Therefore, specific methods for delivery of an oral medication which allow efficient and consistent exposure of the medication is highly desirable.

For example, reversible covalent drug molecules *i.e.,* drugs which contain a Michael acceptor with a second electron withdrawing group, can exhibit poor bioavailability or delayed for systemic absorption when the drug is administered orally which can be manifested by low plasma AUC and/or Cₘₐₓ values resulting in suboptimal efficacy *in vivo.* The poor bioavailability of this new class of drugs can be attributed, in part, to the reactivity of reversible covalent Michael acceptors moiety in these drugs. Accordingly, limiting the exposure of the reversible covalent drugs to the stomach where the combination of low pH and digestive or metabolic enzymes and other sources of thiols occur, a significant increase in systemic exposure of the drug can be obtained.

Furthermore, the expression of metabolizing enzymes, such as cysteine proteases, mucins, transporters and reactive thiol containing molecules in the stomach, such as glutathione, can also contribute to the low oral bioavailability of reversible covalent Michael acceptor- containing drugs (*see, e.g.,* Johnson D. S., et. al., Future Med Chem. 2010 June 1; 2(6):949-964 and Potashman M. H. et al. J. Med. Chem., Vol 52, No. 5. Pgs. 1231-1246). For example, the combination of digestive enzymes, such as the cysteine protease, pepsin, transporters and metabolizing enzymes such as CYP enzymes in the gastric mucosa, can result at low pH in high chemical and/or metabolic transformation of the reversible and irreversible covalent Michael acceptors. Accordingly, by avoiding exposure of the reversible covalent drugs to the stomach where the combination of low pH and digestive or metabolic enzymes and other sources of thiols occur, a significant increase in systemic exposure of these drugs can be obtained. Additionally, avoidance of exposure to the stomach may reduce or altogether eliminate potential adverse side effects of these drugs such as diahrrea and emesis, commonly called vomiting.

### Summary

Compound (I), a BTK inhibitor and currently in development for treatment of autoimmune diseases, is disclosed in Example 31 of the PCT Application No. PCT/US2013058614 filed on September 6, 2013. Although reasonably soluble at greater than 10% (w/v) at low pH, Compound (I) had poor bioavailability as measured by AUC (area under the curve) when it was administered orally (in rats. When a solution of was administered to rats by oral gavage according to Example 11 below the AUC ranged from 668 to 1640 ng^{∗}hr/ml and showed high variability in exposure.

In order to increase bioavailability and decrease variability, the applicants assessed the impact of different site specific gastrointestinal dosing strategies for Compound (I) as determined by AUC as described in Example 11 below. The baseline for comparisons is the AUC obtained when dosing by oral gavageas above. It was surprisingly discovered that administration of Compound (I) intraduodenally (ID) via intraduodenum cannula in above formulation, afforded an approximately 2 fold increase in AUC (994 increased to 1780 ng^{∗}hr/ml) compared to oral dosing of Compound (I) via gastric gavage. With bypass of the initial part of the small intestine by dosing Compound (I) via intrajejunum (IJ) cannula, a 10 to 40 fold increase in AUC was observed as compared to oral gavage (AUC of 14,400 and 26,800 ng^{∗}hr/ml for IJ dosing). In addition, dosing IJ gave less variability in exposure in plasma among animals, especially when compared to the aforementioned oral gavage there was 3 - 10 fold improvement in standard deviation and was more metabolically stable when administered IJ. Differences of this magnitude are not typical for drugs administered orally. Thus, desirable increase in exposure, and a reduction in variability can be achieved by releasing Compound (I) further down the intestinal tract.

Accordingly, among the various aspects of the present disclosure may be noted the provision of certain methods of site specific administration and formulations of Compound (I) or a pharmaceutically acceptable salt thereof for increasing the oral bioavailabilty of Compound (I) or a pharmaceutically acceptable salt thereof.

In one aspect provided is a solid oral dosage form, which is typically a tablet or capsule, comprising:
(A) a core material comprising the (E) isomer, (Z) isomer, or a mixture of (E) and (Z) isomers of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, (S)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile, or a mixture of (R) and (S) isomers of 2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (Compound (I)) having the structure: 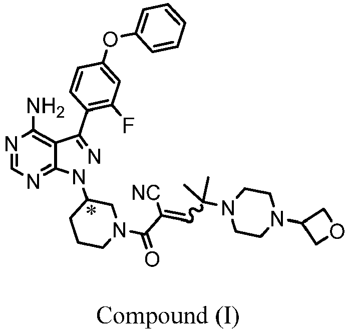
   where *C is a stereochemical center;
   and/or a pharmaceutically acceptable salt thereof;
(B) an enteric coating;
(C) a subcoat below the enteric coating, wherein the subcoat:
   (1) is a water soluble or hydrophilic erodible polymer, said polymer being a low molecular weight polymer selected from hydroxymethyl cellulose (HPMC), hydroxyethyl cellulose, hydroxypropyl cellulose, microcrystalline cellulose, polyvinylpyrrolidones, polysaccharides, a polysaccharide derivative, polyvinyl alcohols, polyethylene glycol (PEG), polypropylene glycol (PPG), and a PEG-PPG block copolymer; or
   (2) comprises a water insoluble composition comprising: (i) particles of a water soluble compound capable of forming channels in the water insoluble composition; or (ii) water insoluble hydrophilic particles which cause swelling of the subcoat when in contact with an aqueous media; and
(D) a pharmaceutically acceptable excipient,
   wherein the solid oral dosage form has an onset of release of Compound (I) and/or the pharmaceutically acceptable salt thereof in the jejuno-ileum portion of the intestine of a mammal after administration; and
   wherein the solid oral dosage form releases at least 80% of Compound (I) and/or the pharmaceutically acceptable salt thereof in 120 minutes in a dissolution vessel comprising an aqueous solution at a pH from 6.4 to 7.4.

In a second aspect, provided is a solid oral dosage form according to the invention for use in a method of treating a disease treatable by the inhibition of BTK in a patient in recognized need thereof, wherein said method comprises administering to the patient a therapeutically effective amount of Compound (I) and/or the pharmaceutically acceptable salt thereof.

In one embodiment of the second aspect, the mammal in need of such disease treatment is suffering from an autoimmune disease, e.g., thrombotic thrombocytopenic purpura, Polyarteritis Nodosa, Cutaneous lupus, cutaneous form of systemic sclerosis (CREST), systemic sclerosis, mixed connective tissue disease, cryoglobulinemia, primary biliary sclerosis, sclerosing cholangitis, AI urticarial, IgA nephropathy, inflammatory bowel disease (such as ulcerative colitis), lupus including lupus nephritis, rheumatoid arthritis, psoriatic arthritis, osteoarthritis, Still's disease, juvenile arthritis, diabetes, myasthenia gravis, Granulomatosis with Polyangiitis, Hashimoto's thyroiditis, Ord's thyroiditis, Graves' disease, Sjogren's syndrome, Sjogren's dry eye, non-Sjogren's dry eye disease, multiple sclerosis, Guillain-Barre syndrome, acute disseminated encephalomyelitis, Addison's disease, opsoclonus-myoclonus syndrome, ankylosing spondylitisis, multiple sclerosis, antiphospholipid antibody syndrome, aplastic anemia, autoimmune hepatitis, coeliac disease, Goodpasture's syndrome, idiopathic thrombocytopenic purpura, optic neuritis, scleroderma, primary biliary cirrhosis, Reiter's syndrome, Takayasu's arteritis, temporal arteritis, autoimmune hemolytic anemia, psoriasis, alopecia universalis, Behcet's disease, chronic fatigue, dysautonomia, endometriosis, interstitial cystitis, neuromyotonia, scleroderma, blistering disease such as pemphigus vulgaris, pemphigoid, and vulvodynia. In another embodiment of the seventh aspect, the disease is rheumatoid arthritis or psoriatic arthritis. In yet another embodiment, the autoimmune disease is lupus, pemphigus vulgaris, Granulomatosis with Polyangiitis, or rheumatoid arthritis.

In another embodiment of the second aspect, the mammal in need of such disease treatment is suffering from a heteroimmune condition or disease, e.g., graft versus host disease, transplantation, transfusion, anaphylaxis, allergy, type I hypersensitivity, allergic conjunctivitis, allergic rhinitis, and atopic dermatitis.

In yet another embodiment of the second aspect, the mammal in need of such disease treatment is suffering from an inflammatory disease, e.g., asthma, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, proctitis, prostatitis, pyelonephritis, rhinitis, salpingitis, sinusitis, stomatitis, synovitis, tendonitis, tonsillitis, uveitis, vaginitis, vasculitis, or vulvitis, preferably asthma or uveitis.

In yet another embodiment of the second aspect, the mammal in need of such disease treatment is suffering from inflammatory skin disease, such as dermatitis, contact dermatitis, eczema, urticaria, rosacea, and scarring psoriatic lesions in the skin, joints, or other tissues or organs.

In yet another embodiment of the second aspect, the mammal in need of such disease treatment is suffering from a cancer. In one embodiment, the cancer is a B-cell proliferative disorder, e.g., diffuse large B cell lymphoma, follicular lymphoma, chronic lymphocytic lymphoma (CLL), chronic lymphocytic leukemia, chromic myleogenous leukemia, B-ALL, Philadelphia chromosome positive B-ALL,B-cell prolymphocytic leukemia, small lymphocytic lymphoma (SLL), multiple myeloma, B-cell non-Hodgkin lymphoma, lymphoplamascytic lymphoma/ Waldenstrom macroglobulinemia, splenic marginal zone lymphoma, plasma cell myeloma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, burkitt lymphoma/leukemia, or lymphomatoid granulomatosis.

In yet another embodiment the second aspect, the mammal in need of such disease treatment is suffering from a thromboembolic disorder, e.g., myocardial infarction, angina pectoris, reocclusion after angioplasty, restenosis after angioplasty, reocclusion after aortocoronary bypass, restenosis after aortocoronary bypass, stroke, transitory ischemia, a peripheral arterial occlusive disorder, pulmonary embolism, or deep venous thrombosis.

In any of the aforementioned embodiments disclosed herein the dosage form of the present disclosure can be administered with another antiflammatory, immunosuppressive, or anticancer agent. In one embodiment, the at least one additional agent is alemtuzumab, arsenic trioxide, asparaginase (pegylated or non-), bevacizumab, cetuximab, platinum-based compounds, such as cisplatin, cladribine, daunorubicin/doxorubicin /idarubicin, irinotecan, fludarabine, 5-fluorouracil, gemtuzamab, methotrexate, paclitaxel, Taxol™, docetaxol, temozolomide, thioguanine, and classes of drugs including hormones (an antiestrogen, an antiandrogen, or gonadotropin releasing hormone analogues, interferons such as alpha interferon, nitrogen mustards such as busulfan or melphalan or mechlorethamine, retinoids such as tretinoin, topoisomerase inhibitors such as irinotecan or topotecan, tyrosine kinase inhibitors such as gefinitinib or imatinib, ofatumumab, bendamustine, rituximab, obinutuzumab, IPI-145, GS-1101, BKM-120, GDC-0941, DGDC-0980, GS-9820, CAL-263, Revlimid®, thalidomide®, pomalidomide®, Velcade®, Kyprolis®, delanzomib, U0126, PD98059, PD184352, PD0325901, ARRY-142886, SB239063, SP600125, BAY 43-9006, wortmannin, Nexavar®, Tarceva®, Sutent®, Tykerb®, Sprycel®, Crizotinib, Xalkori®, or LY294002 or agents to treat signs or symptoms induced by such therapy including allopurinol, filgrastim, granisetron/ondansetron/ palonosetron, dronabinol. When combination therapy is used, the agents can be administered simultaneously or sequentially.

In yet another embodiment the second aspect, the mammal in need of such disease treatment is suffering from thrombotic thrombocytopenic purpura, Polyarteritis Nodosa, Cutaneous lupus, cutaneous form of systemic sclerosis (CREST), systemic sclerosis, mixed connective tissue disease, cryoglobulinemia, primary biliary sclerosis, sclerosing cholangitis, AI urticarial, IgA nephropathy, lupus nephritis, autoimmune hemolytic anemia, Granulomatosis with Polyangiitis, or pemphigus including pemphigus vulgaris.

### BRIEF DESCRIPTION OF THE FIGURES

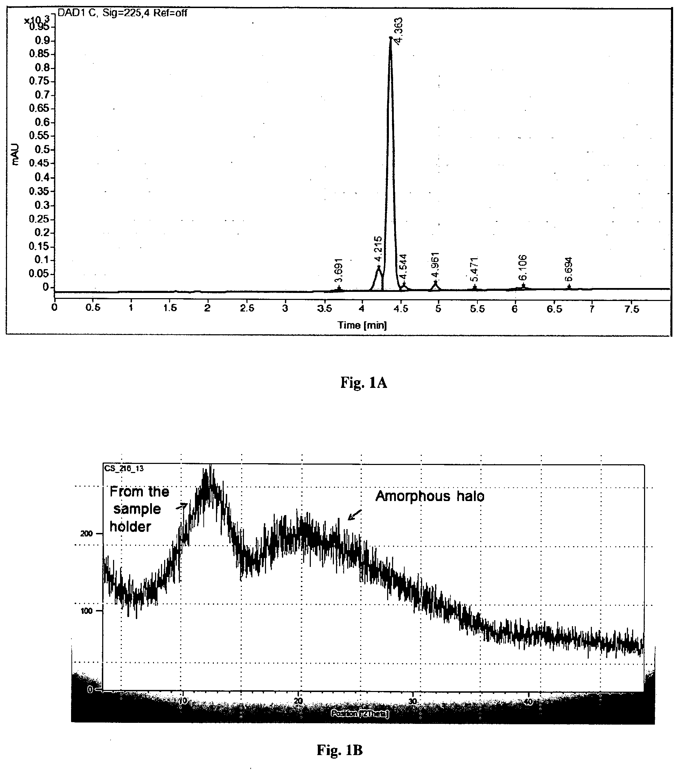

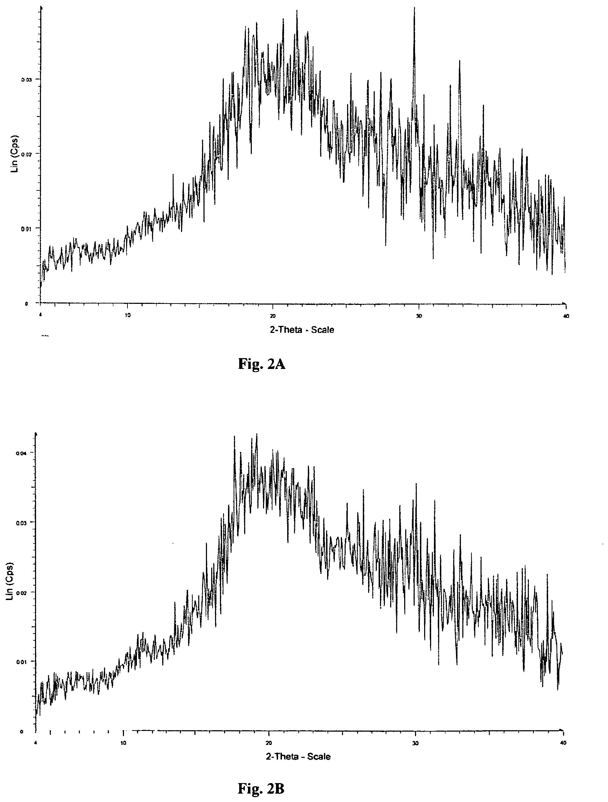

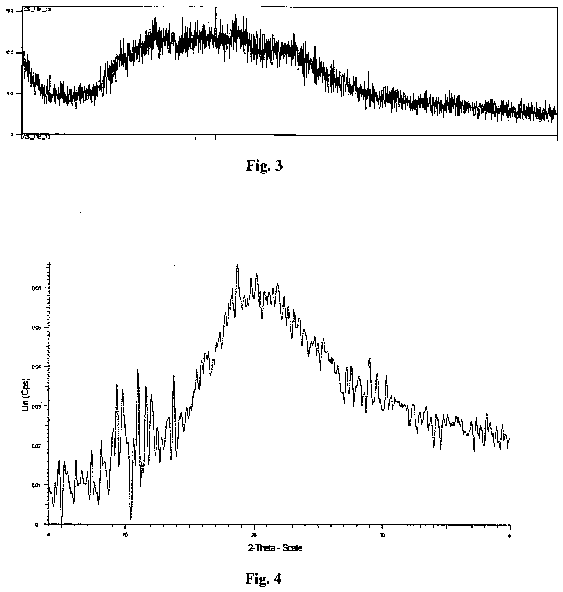

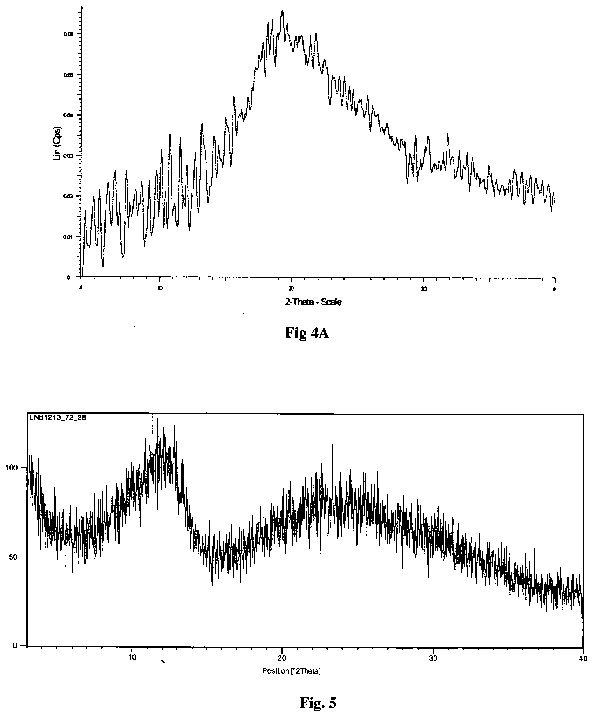

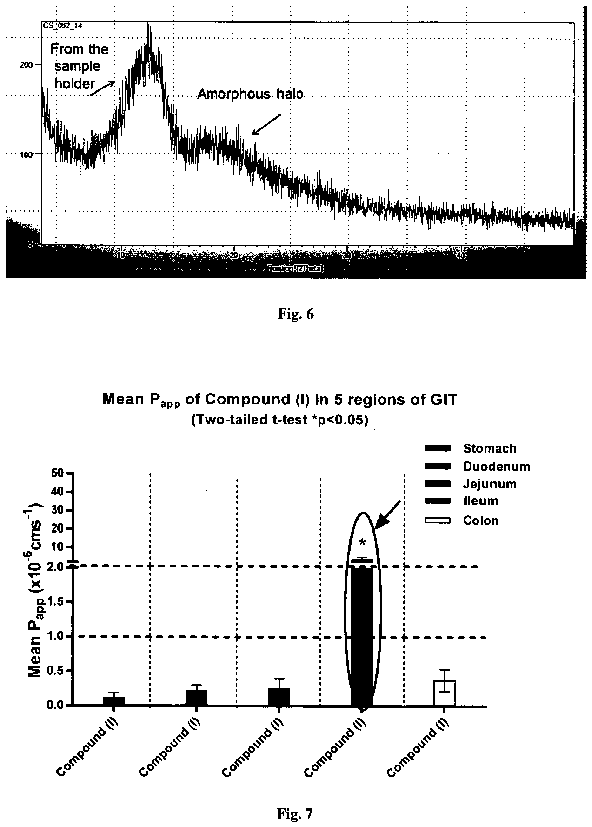

A representative HPLC trace of Compound (I) representing separation of the E and Z isomers of Compound (I) is shown in Figure 1A below. The peak at 4.215 is the Z isomer and the one at 4.363 is the E isomer of Compound (I).
A representative XRPD diffractogram of an amorphous form of Compound (I) having an E/Z ratio of about 9/1 is shown in Figure 1B below.
A representative XRPD diffractogram for hemi-H₂SO₄ salt of Compound (I) having an E/Z ratio of about 9/1 from ethylacetate prepared according to Example 2 is shown in Figure 2A below.
A representative XRPD diffractogram for H₂SO₄ salt from ethylacetate of Compound (I) having an E/Z ratio of about 9/1 prepared according to Example 2 is shown in Figure 2B below.
A representative XRPD diffractogram of an amorphous form of mono-HCl salt of Compound (I) having an E/Z ratio of about 9/1 from ethyl acetate prepared according to Example 3 is shown in Figure 3 below.
A representative XRPD diffractogram for mono-methanesulfonic acid salt of Compound (I) having an E/Z ratio of about 9/1 prepared according to Example 4 from MTBE is shown in Figure 4A.
A representative XRPD diffractogram for di-methanesulfonic salt of Compound (I) having an E/Z ratio of about 9/1 or about 1/9 prepared according to Example 4 from MTBE is shown in Figure 4.
A representative XRPD diffractogram for oxalic acid salt of Compound (I) having an E/Z ratio of about 9/1 prepared according to Example 5 from isopropyl acetate is shown in Figure 5.
A representative XRPD diffractogram for citric acid salt of Compound (I) having an E/Z ratio of about 9/1 prepared according to Example 6 is shown in Figure 6.
Figure 7 depicts the permeablilty of a mixture of E and Z isomers (about 9:1) of Compound (I) in various regions of the GI tract including the stomach, duodenum, ileum, jejunum and colon observed from the study conducted according to Example 12.

### Definitions:

Unless otherwise stated, the following terms used in the specification and claims are defined for the purposes of this Application and have the following meaning. All undefined technical and scientific terms used in this Application have the meaning as commonly understaood by one of ordinary skill in the art to which this invention belongs.

The indefinite article "a" or "an" entity as used herein refers to one or more of that entity; for example, a compound refers to one or more compounds or at least one compound unless stated otherwise. As such, the terms "a" (or "an"), "one or more", and "at least one" can be used interchangeably herein.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 5%.

Compound (I) as used herein means "(E) isomer, (Z) isomer, or a mixture of (E) and (Z) isomers of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, (S)- 2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, or a mixture of (R) and (S) isomers of 2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile" having the structure: 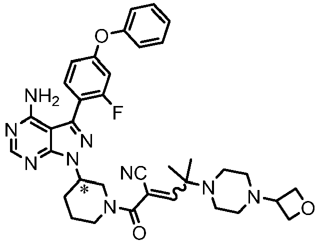
where *C is a stereochemical center;
or a pharmaceutically acceptable salt thereof.

It will be undertood by a person of ordinary skill in the art that when Compound (I) is denoted as (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, it may contain the corresponding (S) enantiomer as an impurity in less than about 1% by wt. Accordingly, when the Compound (I) is denotes as a mixture of (R) and (S) isomers of 2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile it means that the amount of (R) or (S) enantiomer in the mixture is greater than about 1% by wt. Similar analysis applies with when Compound (I) is denoted as the (E) isomer, (Z) isomer or a mixture of (E) and (Z) isomers. Compound (I) or a pharmaceutically acceptable salt thereof may also referred to in the specification as "drug", "active agent", or "a therapeutically active agent".

"Amorphous form" means a solid which does not possess a distinguishable crystal lattice and the molecular arrangement of molecules lack a long range order characteristic of a crystal. In particular amorphous denotes a material that does not show a sharp Bragg diffraction peak.

The term "cellulose derivative" or "polysaccharide derivative" refers to a cellulose polymer or polysaccharide wherein at least a portion of the hydroxyls on the saccharide repeat units have been reacted to form an ether or ester linkage. Examples include and are not limited to hydroxyalkyl celluloses, hydroxyalkyl alkylcelluloses, and carboxyalkyl cellulose esters, such as hydroxypropyl methylcelluloses (hypromelloses or HPMC), hydroxypropylcelluloses (HPC), and the like.

The term "hydrophilic" for purposes of the present disclosure relates to materials that have affinity toward water.

The term "water soluble" for purposes of the present disclosure relates to materials that dissolve to the extent required, in an aqueous media at a pH of from about 1 to about 8, and is not particularly limited.

The term "water swellable" for purposes of the present disclosure relates to materials that are relatively insoluble in water, but which can absorb water.

Suitable hydrophilic materials comprise water soluble or water swellable materials. Examples of such materials include salts, sugars, and polymers such as hydroxyalkyl celluloses, hydroxyalkyl alkylcelluloses, and carboxyalkyl cellulose esters, for example, hydroxypropyl methylcelluloses (hypromelloses or HPMC), hydroxypropylcelluloses (HPC), and combinations comprising one or more of the foregoing materials.

Hydroxypropyl methylcelluloses that are hydrophilic in nature and may be used in the present disclosure are sold in different viscosity grades such as those sold under the brand name Methocel™ available from Dow Chemical Co. Examples of hydroxypropyl methylcelluloses of a low viscosity grade include those available under the brand names Methocel E5, Methocel E-15 LV, Methocel E50 LV, Methocel K100 LV and Methocel F50 LV whose 2% by weight aqueous solutions have viscosities of 5 cP, 15 cP, 50 cP, 100 cP, and 50 cP, respectively. Examples of hydroxypropyl methylcelluloses having medium viscosity include those available under the brand names Methocel E4M and Methocel K4M, both of whose 2% by weight aqueous solutions have a viscosity of 4000 cP. Examples of hydroxypropyl methylcellulose polymers having high viscosity include those available under the brand names Methocel K15M and Methocel K100M whose 2% by weight aqueous solutions have viscosities of 15,000 cP and 100,000 cP, respectively. The hydroxypropyl methylcellulose polymers may be present in the pharmaceutical compositions of the present disclosure in amounts from about 0.1 % to 50% by weight.

The hydroxypropylcellulose polymers that may be used in the present disclosure also include, for example, polymers available under the brand name Klucel™, available from Nippon Soda Co. Hydroxypropylcellulose polymers available under the brand names Klucel EF, Klucel LF, Klucel JF and Klucel GF, whose 2% by weight aqueous solutions have viscosities less than 1000 cP, are examples of low viscosity hydrophilic polymers. A hydroxypropylcellulose polymer available under the brand name Klucel ME whose 2% by weight aqueous solution has a viscosity in the range from 4,000-6,500 cP is a medium viscosity hydrophilic polymer. Hydroxypropyl cellulose polymers available sold as HPC-SL, HPC-L, and HPC-M, whose 2% by weight aqueous solutions have viscosities of 3-6 cP, 6-10 cP, and 150-400 cP, respectively, are examples of low viscosity hydrophilic polymers, while HPC-H has a viscosity of 1 ,000-4000 cP and is an example of a medium viscosity hydrophilic polymer. The hydroxypropylcellulose polymers may be present in an amount from about 0.1 % to 50% by weight.

Water swellable materials suitable for making delayed release dosage forms are compounds that are able to expand when they are exposed to aqueous fluids, such as gastrointestinal fluids. One or more water swellable compounds may be present in a coating and optionally one or more pharmaceutically acceptable excipients.

Suitable compounds which can be used as water swellable substances include, for example, low-substituted hydroxypropyl celluloses, e.g. L-HPC, cross-linked polyvinylpyrrolidones, e.g., PVP-XL, Kollidone™ CL and Polyplasdone™ XL, sodium carboxymethylcellulose, cross-linked sodium carboxymethylcellulose, e.g., Ac-di-sol™ and Primellose™, sodium starch glycolate, e.g., Primojel™, sodium carboxymethylcelluloses, e.g., Nymcel™ ZSB10, sodium carboxymethyl starches, e.g., Explotab™, ion-exchange resins, e.g., Dowex™ or Amberlite™ products, microcrystalline cellulose, e.g., Avicel™ products, starches and pregelatinized starches, e.g., Starch 1500™ and Sepistab ST200™, formalin- casein, e.g., Plas-Vita™, and combinations comprising one or more of the foregoing water swellable substances.

In some embodiments, hydrophilic materials include polyalkylene oxides, polysaccharide gums, and crosslinked polyacrylic acids. Suitable polyalkylene oxides, such as linear polymers of unsubstituted ethylene oxide, include Polyox™ products from The Dow Chemical Company, U.S., having molecular weights about 100,000-7,000,000. Other useful polyalkylene oxide polymers are made from propylene oxide, or mixtures of ethylene oxide and propylene oxide.

Polysaccharide gums, both natural and modified (semi-synthetic), can be used. Examples are dextran, xanthan gum, gellan gum, welan gum and rhamsan gum.

Crosslinked polyacrylic acids that can be used include those having properties similar to those described above for alkyl-substituted cellulose and polyalkylene oxide polymers. Useful crosslinked polyacrylic acids include those with viscosities about 4,000 to about 40,000 cP (for a 1 % aqueous solution at 25°C). Three specific examples are CARBOPOL™ grades 971 P, 974P, and 934P (sold by The Lubrizol Corporation, Cleveland, Ohio, USA). Further examples are polymers known as WATER LOCK™, which are starch/acrylate/acrylamide copolymers available from Grain Processing Corporation, Muscatine, Iowa, USA.

The hydrophilicity and water swellability of these polymers cause the subcoat to swell in size after oral administration, due to ingress of water. The release rate of an active agent from the subcoat is primarily dependent upon the rate of water inhibition and the rate at which the active agent dissolves and diffuses from the swollen polymer, which in turn is related to the solubility and dissolution rate of the active agent, the active agent particle size, and/or the active agent concentration in the dosage form.

Suitable "hydrophobic" materials are water-insoluble neutral or synthetic waxes, fatty alcohols such as lauryl, myristyl, stearyl, cetyl, or cetostearyl alcohol, fatty acids and derivatives thereof, including fatty acid esters such as such as glyceryl monostearate, glycerol monooleate, acetylated monoglycerides, stearin, palmitin, laurin, myristin, cetyl esters wax, glyceryl palmitostearate, glyceryl behenate, hydrogenated castor oils, cottonseed oils, fatty acid glycerides (mono-, di-, and tri-glycerides), hydrogenated fats, hydrocarbons, normal waxes, stearic acid, stearyl alcohol, materials having hydrocarbon backbones, and combinations comprising one or more of the foregoing materials. Suitable waxes include, but are not limited to, beeswax, Glycowax® (a N,N'-distearoylethyelenediamine, from Lonza), castor wax, carnauba wax, and wax-like substances.

The term "immediate release dosage form" as used herein refers to a dosage formulation, in liquid or solid form, which releases drug in the stomach and does not have a protective coating to delay contact of the drug with the intestinal mucosa. An aestheric or taste masking coating may be included in the "immediate release dosage form".

The term "intraduodenal release dosage form" as used herein refers to a dosage formulation, in liquid or solid form, which begins to release drug in the duodenum.

"Mammal" as used herein means domesticated animals (such as dogs, cats, and horses), and humans. In one embodiment, mammal is a human.

A "pharmaceutically acceptable salt" of as used herein means an acid addition salt that is pharmaceutically acceptable and that possesses the desired pharmacological activity of the compound from which the salt is made (hereafter, sometimess referred to as "parent compound"). Such salts include salts, formed with inorganic acids such as hydrochloric acid, hydrobromic acid, sulfuric acid, phosphoric acid, and the like; or formed with organic acids such as formic acid, acetic acid, propionic acid, hexanoic acid, lactic acid, malonic acid, succinic acid, malic acid, maleic acid, fumaric acid, tartaric acid, citric acid, benzoic acid, mandelic acid, methanesulfonic acid, ethanesulfonic acid, 1,2-ethanedisulfonic acid, benzenesulfonic acid, 4-toluenesulfonic acid, and the like.

A "pharmaceutically acceptable carrier or excipient" means a carrier or an excipient that is useful in preparing a pharmaceutical composition; is generally safe, and neither biologically nor otherwise undesirable, and includes a carrier or an excipient that is acceptable for mammalian pharmaceutical use.

"Treating" or "treatment" of a disease includes:
(1) inhibiting the disease, i.e., arresting or reducing the development of the disease or its clinical symptoms; or
(2) relieving the disease, i.e., causing regression of the disease or its clinical symptoms.

A "therapeutically effective amount" means the amount of Compound (I) that, when administered to a mammal in need or recognized need of treatment for treating a disease, is sufficient to effect such treatment for the disease. The "therapeutically effective amount" will vary depending on the compound, the disease and its severity, and the age, weight, etc., of the mammal to be treated.

"Substantially pure" as used herein refers to a compound (or salt thereof) such as Compound I (or salt thereof) wherein at least about 70% by weight of the compound (or salt thereof) is present as the given solid state form. For example, the phrase "amorphous form of a salt of Compound (I) (or a salt thereof) in substantially pure amorphous form " refers to a solid state form of Compound 1 (or a salt thereof) wherein more than about 70% by weight of Compound (or a salt thereof) is an amorphous form with the remaining present in a crystalline form. In one embodiment, such compositions contain at least about 80% by weight of Compound (I) (or a salt thereof) in amorphous form. In another embodiment at least about 85% by weight of Compound (I) (or a salt thereof) is in amorphous form. In yet another embodiment, at least about 90% by weight of Compound (I) (or a salt thereof) is in amorphous form. In yet another embodiment, at least about 95% by weight of Compound (I) (or a salt thereof) is in amorphous form. In yet another embodiment, at least about 97% by weight or about 98% by weight of Compound (I) (or a salt thereof) is in amorphous form. In yet another embodiment, at least about 99% by weight of Compound (I) is in amorphous form. The relative amounts of crystalline and/or amorphous forms in a solid mixture can be determined by well known in the art. For example, X-Ray diffraction provides a convenient and practical means for quantitative determination of the relative amounts of crystalline and/or amorphous forms in a solid mixture. X-Ray diffraction is adaptable to quantitative applications because the intensities of the diffraction peaks of a given compound in a mixture are proportional to the fraction of the corresponding powder in the mixture. Although all salts of Compound (I) are amorphous, if any crystalline form of Compound (I) (or a salt thereof) is present in a mixture, percent composition of crystalline Compound (I) (or a salt thereof) in an unknown composition can be determined. Preferably, the measurements are made on solid powder of Compound (I) (or a salt thereof). The X-Ray powder diffraction patterns of an unknown composition may be compared to known quantitative standards containing pure crystalline forms, if any, of Compound (I) (or a salt thereof) to identify the percent ratio of a particular crystalline form. If an amorphous form is the major fraction of the composition, the amount may be further compared to the total weight of the solid subject to analysis. This is done by comparing the relative intensities of the peaks from the diffraction pattern of the unknown solid powder composition with a calibration curve derived from the X-Ray diffraction patterns of pure known samples. The curve can be calibrated based on the X-Ray powder diffraction pattern for the strongest peak from a pure sample of crystalline forms of Compound (I) (or a salt thereof). The calibration curve may be created in a manner known to those of skill in the art. For example, five or more artificial mixtures of crystalline forms of Compound (I) (or a salt thereof), at different amounts, may be prepared. In a non-limiting example, such mixtures may contain, 2%, 5%, 7%, 8%, and 10% of Compound (I) (or a salt thereof) for each crystalline form. Then, X-Ray diffraction patterns are obtained for each artificial mixture using standard X-Ray diffraction techniques. Slight variations in peak positions, if any, may be accounted for by adjusting the location of the peak to be measured. The intensities of the selected characteristic peak(s) for each of the artificial mixtures are then plotted against the known weight percentages of the crystalline form. The resulting plot is a calibration curve that allows determination of the amount of the crystalline forms of Compound (I) (or a salt thereof) in an unknown sample. For the unknown mixture of crystalline and amorphous forms of Compound (I) (or a salt thereof), the intensities of the selected characteristic peak(s) in the mixture, relative to an intensity of this peak in a calibration mixture, may be used to determine the percentage of the given crystalline form in the composition, with the remainder determined to be the amorphous material. The overall crystallinity may be determined as follows: % Crystallinity=(C/A+C-B) X 100, where C is area under crystalline peaks, A is area under amorphous halo, and B is background noise due to air scattering, fluorescence, etc.

The term "channel" as used herein refers to a pathway in a coating or subcoating that allows influx of at least water into the dosage form In one embodiment, the influx of water into the dosage form creates osmotic pressure inside the dosage form and causes efflux of the drug from the dosage form. In another embodiment, the osmotic pressure causes rupture of the subcoat or coat to release the drug from the dosage form. Materials that can form channels or pores in a coating are well known in the art. They can be organic or inorganic, and include materials that can be dissolved, extracted or leached from the coating, in the environment of use.

AUC ("Area under the curve") is one of the parameters used to assess bioavailability of a drug molecule.

It refers to the area under a curve (i.e., the integral) of a plot of concentration of drug in blood plasma against time. The AUC (from zero to infinity) for a single dose represents the total drug exposure over time and is proportional to the total amount of drug from that single dose absorbed by the body (*i.e.,* the total amount of unchanged drug that reaches the blood circulation) assuming linear pharmacokinetics.

The solid dosage forms described herein comprise an enteric coating, i.e., as an oral dosage form of a pharmaceutical composition as described herein which utilizes an enteric coating to effect the release of the compound in the intestine of the gastrointestinal tract. An "enterically coated" drug or tablet refers to a drug or tablet that is coated with a substance - i.e., with an "enteric coating" - that remains intact in the stomach but dissolves and releases the drug once the intestine (in one embodiment small intestine) is reached. As used herein "enteric coating", is a material, such as a polymer material or materials which encase the therapeutically active agent either as a dosage form or as particles. Typically, a substantial amount or all of the enteric coating material is dissolved before the therapeutically active agent is released from the dosage form, so as to achieve delayed dissolution of the therapeutically active agent in the intestine. Enteric coatings are discussed, for example, Loyd, V. Allen, Remington: The Science and Practice of Pharmacy, Twenty-first Ed., (Pharmaceutical Press, 2005; and P.J. Tarcha, Polymers for Controlled Drug Delivery, Chapter 3, CRC Press, 1991. Methods for applying enteric coatings to pharmaceutical compositions are well known in the art, and include for example, U.S. Patent Publication No. 2006/0045822.

The dosage form may be a compressed or molded or extruded tablet (coated with an enteric coating or uncoated) containing granules, powder, pellets, beads or particles of Compound (I) or a pharmaceutically acceptable salt thereof (or any embodiments thereof) optionally admixed with other excipient(s), which are themselves coated with an enteric coating or uncoated provided at least the tablet and/or the granules, powder, pellets, beads or particles of Compound (I) is coated. The oral dosage form may also be a capsule (coated with an enteric coating or uncoated) containing pellets, beads or granules of the compound of Formula (I) or a pharmaceutically acceptable salt thereof (or any embodiments thereof) optionally admixed with other excipient(s), which are themselves coated with an enteric coating or uncoated provided at least the capsule and/or the granules, powder, pellets, beads or particles of Compound (I) is coated. Some examples of coatings that were originally used as enteric coatings are beeswax and glyceryl monostearate; beeswax, shellac and cellulose; and cetyl alcohol, mastic and shellac as well as shellac and stearic acid (U.S. Pat. No. 2,809,918); polyvinylacetate and ethyl cellulose (U.S. Pat. No. 3,835,221). More recently, the enteric coatings used are neutral copolymers of polymethacrylic acid esters (Eudragit L30D). (F. W. Goodhart et al, Pharm. Tech., p. 64-71, April, 1984); copolymers of methacrylic acid and methacrylic acid methyl ester (Eudragit S), or a neutral copolymer of polymethacrylic acid esters containing metallic stearates (see Mehta et al U.S. Pat. Nos. 4,728,512 and 4,794,001), cellulose acetate succinate, and hypromellose phthalate.

Any anionic polymer exhibiting a pH-dependent solubility profile can be used as an enteric coating in the methods and compositions described herein to achieve site specific delivery to the intestine. In one embodiment, site specific delivery is to jejunum and/or ileum. In some embodiments the polymers described herein are anionic carboxylic polymers. In other embodiments, the polymers and compatible mixtures thereof, and some of their properties, include, but are not limited to:
Shellac: Also called purified lac, it is a refined product obtained from the resinous secretion of an insect. This coating dissolves in media of pH>7;
Acrylic polymers: The performance of acrylic polymers (primarily their solubility in biological fluids) can vary based on the degree and type of substitution. Examples of suitable acrylic polymers include methacrylic acid copolymers and ammonium methacrylate copolymers. The Eudragit series L, S, and RS (manufactured by Rohm Pharma and known as Evonik®) are available as solubilized in organic solvent, aqueous dispersion, or dry powders. The Eudragit series RL, NE, and RS are insoluble in the gastrointestinal tract but are permeable and are used primarily for colonic targeting. The Eudragit series L, L-30D and S are insoluble in stomach and dissolve in the intestine;
Cellulose Derivatives: Examples of suitable cellulose derivatives are: ethyl cellulose; reaction mixtures of partial acetate esters of cellulose with phthalic anhydride. The performance can vary based on the degree and type of substitution. Cellulose acetate phthalate (CAP) dissolves in pH>6. Aquateric (FMC) is an aqueous based system and is a spray dried CAP pseudolatex with particles <1 µm. Other components in Aquateric can include pluronics, Tweens, and acetylated monoglycerides. Other suitable cellulose derivatives include; cellulose acetate tritnellitate (Eastman); methylcellulose (Pharmacoat, Methocel); hydroxypropylmethyl cellulose phthalate (HPMCP); hydroxypropylmethyl cellulose succinate (HPMCS); and hydroxypropylmethylcellulose acetate succinate (HPMCAS e.g., AQOAT (Shin Etsu)). The performance can vary based on the degree and type of substitution. For example, HPMCP such as, HP-50, HP-55, HP-55S, HP-55F grades are suitable. The performance can vary based on the degree and type of substitution. For example, suitable grades of hydroxypropylmethylcellulose acetate succinate include, but are not limited to, AS-LG (LF), which dissolves at pH 5, AS-MG (MF), which dissolves at pH 5.5, and AS-HG (HF), which dissolves at higher pH. These polymers are offered as granules, or as fine powders for aqueous dispersions;
Poly Vinyl Acetate Phthalate (PVAP): PVAP dissolves in pH>5, and it is much less permeable to water vapor and gastric fluids. Detailed description of above polymers and their pH-dependent solubility can be found at in the article titled "Enteric coated hard gelatin capsules" byProfessor Karl Thoma and Karoline Bechtold at http://pop.www.capsugel.com/media/library/enteric-coated-hard-gelatin-capsules.pdf.

In one embodiment the enteric coating is made from acrylic acid, methacrylic acid or ethacrylic acid polymers or copolymers, cellulose acetate (and its succinate and phthalate derivatives), hydroxypropyl methyl cellulose phthalate, polyvinyl acetate phthalate, hydroxyethyl ethyl cellulose phthalate, cellulose acetate tetrahydrophtalate, acrylic resin or shellac. In another embodiment the polymer is chosen from cellulose acetate phthalate (CAP; dissolves above pH 6), polyvinyl acetate phthalate (PVAP, disintegrates at pH 5), hydroxypropyl methyl cellulose phthalate (HPMCP, grade HP50 disintegrates at pH 5 and HP50 disintegrates at 5.5), methylacrylic acid copolymers (Eudragit L 100 and L12.5 disintegrate between about 6 and about 7, Eudragit L-30 and L100-55 disintegrate at pH greater than 5.5 and Eudragit S100, S12.5 and FS 30D disintegrate at pH greater than 7).

In some embodiments, the coating can, and usually does, contain a plasticizer and possibly other coating excipients such as colorants, talc, and/or magnesium stearate, which are well known in the art. Suitable plasticizers include triethyl citrate (Citroflex 2), triacetin (glyceryl triacetate), acetyl triethyl citrate (Citroflec A2), Carbowax 400 (polyethylene glycol 400), diethyl phthalate, tributyl citrate, acetylated monoglycerides, glycerol, fatty acid esters, propylene glycol, and dibutyl phthalate. In particular, anionic carboxylic acrylic polymers usually will contain 10-25% by weight of a plasticizer, especially dibutyl phthalate, polyethylene glycol, triethyl citrate and triacetin.

Conventional coating techniques such as fluid bed or Wurster coaters, or spray or pan coating are employed to apply coatings. The coating thickness must be sufficient to ensure that the oral dosage form remains intact until the desired site of delivery in the intestinal tract is reached. The amount of plasticizer is optimized for each enteric coating layer and the applied amount of said polymer(s), in such a way that the mechanical properties, i.e. flexibility and hardness of the enteric coating layer(s), for instance exemplified as Vickers hardness, are adjusted so that if a tablet is desired the acid resistance of the pellets covered with enteric coating layer(s) does not decrease significantly during compression of pellets into tablets. The amount of plasticizer is usually above 5% by weight of the enteric coating layer polymer(s), (In one embodiment the amount of plasticizer is 15-50%. In another embodiment the amount of plasticizer is 20-50%). The maximum thickness of the applied enteric coating is normally only limited by processing conditions and the desired dissolution profile.

Colorants, surfactants, anti-adhesion agents, antifoaming agents, lubricants (e.g., carnuba wax or PEG) and other additives may be added to the coatings besides plasticizers to solubilize or disperse the coating material, and to improve coating performance and the coated product. To accelerate the dissolution of the enteric coat, a half-thickness, double coat of enteric polymer (for instance, Eudragit L30 D-55) may be applied, and the inner enteric coat may have a buffer up to pH 6.0 in the presence of 10% citric acid, followed by a final layer of standard Eudragit L 30 D-55. Applying two layers of enteric coat, each half the thickness of a typical enteric coat, Liu and Basit were able to accelerate enteric coating dissolution compared to a similar coating system applied, unbuffered, as a single layer (Liu, F. and Basit, A. Journal of Controlled Release. 147 (2010) 242-245.). The intactness of the enteric coating may be measured, for example, by the degradation of the drug within the micropellets. The enteric coated dosage forms or pellets may be tested in dissolution testing first in gastric fluid and separately in intestinal fluid as described in USP to determine its function. Additives such as dispersants, colorants, pigments polymers (*e.g.* poly(ethylacrylate, methylmethacrylate), anti-tacking and anti-foaming agents may also be included into the enteric coating layer(s). Other compounds may be added to increase film thickness and to decrease diffusion of acidic gastric juices into the acid susceptible material.

Formulations disclosed herein contain one or more pharmaceutically acceptable excipient(s) such as e.g., binders, surfactants, diluents, buffering agents, antiadherents, glidants, hydrophilic or hydrophobic polymers, retardants, stabilizing agents or stabilizers, disintegrants or superdisintegrants, dispersants, antioxidants, antifoaming agents, fillers, flavors, colorants, lubricants, sorbents, preservatives, plasticizers, or sweeteners, or mixtures thereof, which facilitate processing of the drug molecule (or embodiments thereof disclosed herein) or a pharmaceutically acceptable salt thereof into preparations which can be used pharmaceutically. The pharmaceutically acceptable excipients can be in the coating and/or the core. Any of the well-known techniques and excipients may be used as suitable and as understood in the art, *see* for example, Remington: The Science and Practice of Pharmacy, Twenty-first Ed., (Pharmaceutical Press, 2005); Liberman, H. A., Lachman, L., and Schwartz, J.B. Eds., Pharmaceutical Dosage Forms, Vol. 1-2 Taylor & Francis 1990; and R.I. Mahato, Ansel's Pharmaceutical Dosage Forms and Drug Delivery Systems, Second Ed. (Taylor & Francis, 2012).

In some embodiments, the formulations may include one or more pH adjusting agents or buffering agents, for example, acids such as acetic, boric, citric, lactic, phosphoric and hydrochloric acids; bases such as sodium hydroxide, sodium phosphate, sodium borate, sodium citrate, sodium acetate, sodium lactate and *tris*-hydroxymethylaminomethane; and buffers such as citrate/dextrose, sodium bicarbonate, ammonium chloride, and the like. Such acids, bases and buffers are included in an amount required to maintain pH of the composition in an acceptable range.

In some embodiments, the formulations may also include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

In some embodiments, the formulations may also include one or more antifoaming agents to reduce foaming during processing which can result in coagulation of aqueous dispersions, bubbles in the finished film, or generally impair processing. Exemplary anti-foaming agents include silicon emulsions or sorbitan sesquoleate.

In some embodiments, the formulations may also include one or more antioxidants, such as non-thiol antioxidants, for example, butylated hydroxytoluene (BHT), sodium ascorbate, ascorbic acid, and tocopherol. In certain embodiments, antioxidants enhance chemical stability where required.

In some embodiments, the formulations may also include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

In some embodiments, the formulations may also include one or more binders. Binders impart cohesive qualities and include, *e.g.,* alginic acid and salts thereof; cellulose derivatives such as carboxymethylcellulose, methyl cellulose (*e.g.,* Methocel®), hydroxypropylmethylcellulose, hydroxyethylcellulose, hydroxypropylcellulose (*e.g.,* Klucel®), ethylcellulose (*e.g.,* Ethocel®), and microcrystalline cellulose (*e.g.,* Avicel®); microcrystalline dextrose; amylose; magnesium aluminum silicate; polysaccharide acids; bentonites; gelatin; polyvinyl-pyrrolidone/vinyl acetate copolymer; crosspovidone; povidone; starch; pregelatinized starch; tragacanth, dextrin, maltodextrin,a sugar, such as sucrose (*e.g.,* Dipac®), glucose, dextrose, molasses, mannitol, sorbitol, xylitol (*e.g.,* Xylitab®), and lactose; a natural or synthetic gum such as acacia, tragacanth, ghatti gum mucilage of isapol husks, polyvinylpyrrolidone (*e.g.,* Polyvidone® CL, Kollidon® CL, Polyplasdone® XL-10), larch arabogalactan, Veegum®, polyethylene glycol, polyethylene oxide, waxes, sodium alginate, and the like.

In general, binder levels of about 10 to about 70% are used in powder-filled gelatin capsule formulations. Binder usage level in tablet formulations varies whether direct compression, wet granulation, roller compaction, or usage of other excipients such as fillers which itself can act as moderate binder. Formulators skilled in art can determine the binder level for the formulations, but binder usage level of up to 70% in tablet formulations is common.

In some embodiments, the formulations may also include dispersing agents and/or viscosity modulating agents. Dispersing agents and/or viscosity modulating agents include materials that control the diffusion and homogeneity of a drug through liquid media or a granulation method or blend method. In some embodiments, these agents also facilitate the effectiveness of a coating or eroding matrix. Exemplary diffusion facilitators/dispersing agents include, *e.g.,* hydrophilic polymers, electrolytes, Tween® 20, 60 or 80, PEG, polyvinylpyrrolidone (PVP; commercially known as Plasdone®), and the carbohydrate-based dispersing agents such as, for example, hydroxypropyl celluloses (*e.g.,* HPC, HPC-SL, and HPC-L), hydroxypropyl methylcelluloses (*e.g.,* HPMC K100, RPMC K4M, HPMC K15M, and HPMC K100M), carboxymethylcellulose sodium, methyl cellulose, triethylcellulose, hydroxyethyl-cellulose, hydroxypropyl-cellulose, hydroxypropylmethylcellulose phthalate, hydroxypropyl-methylcellulose acetate stearate (HPMCAS), noncrystalline cellulose, magnesium aluminum silicate, triethanolamine, polyvinyl alcohol (PVA), vinyl pyrrolidone/vinyl acetate copolymer (S630), 4-(1,1,3,3-tetramethylbutyl)-phenol polymer with ethylene oxide and formaldehyde (also known as tyloxapol), poloxamers (*e.g.,* Pluronics® F68, F88, and F108, which are block copolymers of ethylene oxide and propylene oxide); and poloxamines (e.g., Tetronic® 908, also known as Poloxamine® 908, which is a tetrafunctional block copolymer derived from sequential addition of propylene oxide and ethylene oxide to ethylenediamine (BASF Corporation, Parsippany, N.J.)), polyvinylpyrrolidone K12, polyvinylpyrrolidone K17, polyvinylpyrrolidone K25, or polyvinylpyrrolidone K30, polyvinylpyrrolidone/vinyl acetate copolymer (S-630), polyethylene glycol, e.g., the polyethylene glycol can have a molecular weight of about 300 to about 6000, or about 3350 to about 4000, or about 7000 to 5400, sodium carboxymethylcellulose, methylcellulose, polysorbate-80, sodium alginate, gums, such as, *e.g.,* gum tragacanth and gum acacia, guar gum, xanthans, including xanthan gum, sugars, cellulosics, such as, e.g., sodium carboxymethylcellulose, methylcellulose, sodium carboxymethylcellulose, polysorbate-80, sodium alginate, polyethoxylated sorbitan monolaurate, polyethoxylated sorbitan monolaurate, povidone, carbomers, polyvinyl alcohol (PVA), alginates, chitosans and combinations thereof.

In some embodiments, the formulations may also include one or more "diluents" which refers to chemical compounds that are used to dilute the compound of interest prior to delivery. Diluents can also be used to stabilize compounds because they can provide a more stable environment Salts dissolved in buffered solutions (which also can provide pH control or maintenance) are utilized as diluents in the art, including, but not limited to a phosphate buffered saline solution. In certain embodiments, diluents increase bulk of the composition to facilitate compression or create sufficient bulk for homogenous blend for capsule filling. Such compounds include e.g., lactose, starch, mannitol, sorbitol, dextrose, microcrystalline cellulose such as Avicel®; dibasic calcium phosphate, dicalcium phosphate dihydrate; tricalcium phosphate, calcium phosphate; anhydrous lactose, spray-dried lactose; pregelatinized starch, compressible sugar, such as Di-Pac® (Amstar); hydroxypropyl-methylcellulose, hydroxypropylmethylcellulose acetate stearate, sucrose-based diluents, confectioner's sugar; monobasic calcium sulfate monohydrate, calcium sulfate dihydrate; calcium lactate trihydrate, dextrates; hydrolyzed cereal solids, amylose; powdered cellulose, calcium carbonate; glycine, kaolin; mannitol, sodium chloride; inositol, bentonite, and the like.

In some embodiments, the formulation may contain surface active agents or surfactants are long chain molecules that can accumulate at hydrophilic/hydrophobic (water/oil) interfaces and lower the surface tension at the interface. As a result they can stabilise an emulsion. In some embodiments, the surfactant may comprise: Tween® (polyoxyethylene sorbate) family of surfactants, Span® (sorbitan long chain carboxylic acid esters) family of surfactants, Pluronic® (ethylene or propylene oxide block copolymers) family of surfactants, Labrasol®, Labrafil® and Labrafac®(each polyglycolyzed glycerides) families of surfactants, sorbitan esters of oleate, stearate, laurate or other long chain carboxylic acids, poloxamers (polyethylene-polypropylene glycol block copolymers or Pluronic®), other sorbitan or sucrose long chain carboxylic acid esters, mono and diglycerides, PEG derivatives of caprylic/capric triglycerides and mixtures thereof or mixture of two or more of the above. In some embodiments the surfactant phase may comprise a mixture of Polyoxyethylene (20) sorbitan monooleate (Tween 80®) and sorbitan monooleate (Span 80®).

In some embodiments, the formulations may also include one or more "disintegrant" which includes both the dissolution and dispersion of the dosage form when contacted with gastrointestinal fluid. "Disintegration agents or disintegrants" facilitate the breakup or disintegration of a substance. Examples of disintegration agents include a starch, e.g., a natural starch such as corn starch or potato starch, a pregelatinized starch such as National 1551 or sodium starch glycolate such as Promogel® or Explotab®, a cellulose such as a wood product, methyl crystalline cellulose, *e.g.,* Avicel®, Avicel® PH101, Avicel® PH 102, Avicel® PH105, Elceme® PI00, Emcocel®, Vivacel®, and Solka-Floc®, methylcellulose, croscarmellose, or a cross-linked cellulose, such as cross-linked sodium carboxymethylcellulose (Ac-Di-Sol®), cross-linked carboxymethylcellulose, or cross-linked croscarmellose, a cross-linked starch such as sodium starch glycolate, a cross-linked polymer such as crosspovidone, a cross-linked polyvinylpyrrolidone, alginate such as alginic acid or a salt of alginic acid such as sodium alginate, a clay such as Veegum® HV (magnesium aluminum silicate), a gum such as agar, guar, locust bean, Karaya, pectin, or tragacanth, sodium starch glycolate, bentonite, a natural sponge, a surfactant, a resin such as a cation-exchange resin, citrus pulp, sodium lauryl sulfate, sodium lauryl sulfate in combination starch, and the like.

In some embodiments, the formulations may also include erosion facilitators. "Erosion facilitators" include materials that control the erosion of a particular material in gastrointestinal fluid. Erosion facilitators are generally known to those of ordinary skill in the art. Exemplary erosion facilitators include, *e.g.,* hydrophilic polymers, electrolytes, proteins, peptides, and amino acids.

In some embodiments, the formulations may also include one or more filling agents which include compounds such as lactose, calcium carbonate, calcium phosphate, dibasic calcium phosphate, calcium sulfate, microcrystalline cellulose, cellulose powder, dextrose, dextrates, dextran, starches, pregelatinized starch, sucrose, xylitol, lactitol, mannitol, sorbitol, sodium chloride, polyethylene glycol, and the like.

In some embodiments, the formulations may also include one or more flavoring agents and/or "sweeteners" *e.g.,* acacia syrup, acesulfame K, alitame, anise, apple, aspartame, banana, Bavarian cream berry, black currant, butterscotch, calcium citrate, camphor, caramel, cherry, cherry cream chocolate, cinnamon, bubble gum, citrus, citrus punch, citrus cream, cotton candy, cocoa, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, monoammonium glyrrhizinate, maltol, mannitol, maple, marshmallow, menthol, mint cream, mixed berry, neohesperidine DC, neotame, orange, pear, peach, peppermint, peppermint cream, powder, raspberry, root beer, rum, saccharin, safrole, sorbitol, spearmint, spearmint cream, strawberry, strawberry cream, stevia, sucralose, sucrose, sodium saccharin, saccharin, aspartame, acesulfame potassium, mannitol, talin, sylitol, sucralose, sorbitol, Swiss cream, tagatose, tangerine, thaumatin, tutti fruitti, vanilla, walnut, watermelon, wild cherry, wintergreen, xylitol, or any combination of these flavoring ingredients, *e.g.,* anise-menthol, cherry-anise, cinnamon-orange, cherry-cinnamon, chocolate-mint, honey-lemon, lemon-lime, lemon-mint, menthol-eucalyptus, orange-cream, vanilla-mint, and mixtures thereof.

In some embodiments, the formulations may also include one or more lubricants and glidants which are compounds that prevent, reduce or inhibit adhesion or friction of materials. Exemplary lubricants include, *e.g.,* stearic acid, calcium hydroxide, talc, sodium stearyl fumerate, a hydrocarbon such as mineral oil, or hydrogenated vegetable oil such as hydrogenated soybean oil, higher fatty acids and their alkali-metal and alkaline earth metal salts, such as aluminum, calcium, magnesium, zinc, stearic acid, sodium stearates, glycerol, talc, waxes, boric acid, sodium benzoate, sodium acetate, sodium chloride, leucine, a polyethylene glycol (*e.g.,* PEG4000) or a methoxypolyethylene glycol such as Carbowax®, sodium oleate, sodium benzoate, glyceryl behenate, polyethylene glycol, magnesium or sodium lauryl sulfate, colloidal silica such as Syloid®, Cab-O-Sil®, a starch such as corn starch, silicone oil, a surfactant, and the like.

In some embodiments, the formulations may also include one or more solubilizers which include compounds such as triacetin, triethylcitrate, ethyl oleate, ethyl caprylate, sodium lauryl sulfate, sodium doccusate, vitamin E TPGS, dimethylacetamide, N-methylpyrrolidone, N-hydroxyethylpyrrolidone, polyvinylpyrrolidone, hydroxypropylmethyl cellulose, hydroxypropyl cyclodextrins for example Captisol®, ethanol, n-butanol, isopropyl alcohol, cholesterol, bile salts, polyethylene glycol 200-600, glycofurol, transcutol, propylene glycol, and dimethyl isosorbide and the like. In one embodiment, the solubilizer is vitamin E TPGS and/or Captisol®.

In some embodiments, the formulations may also include one or more suspending agents which include compounds such as polyvinylpyrrolidone, *e.g.,* polyvinylpyrrolidone K112, polyvinylpyrrolidone K17, polyvinylpyrrolidone K25, or polyvinylpyrrolidone K30, vinyl pyrrolidone/vinyl acetate copolymer (S630), polyethylene glycol, *e.g.,* the polyethylene glycol can have a molecular weight of about 300 to about 6000, or about 3350 to about 4000, or about 7000 to about 5400, sodium carboxymethylcellulose, methylcellulose, hydroxypropylmethylcellulose, hydroxymethylcellulose acetate stearate, polysorbate-80, hydroxyethylcellulose, sodium alginate, gums, such as, *e.g.,* gum tragacanth and gum acacia, guar gum, xanthans, including xanthan gun, sugars, cellulosics, such as, *e.g.,* sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated sorbitan monolaurate, povidone and the like.

In certain embodiments, the formulations may also include one or more surfactants which include compounds such as sodium lauryl sulfate, sodium docusate, Tween 60 or 80, triacetin, vitamin E TPGS, sorbitan monooleate, polyoxyethylene sorbitan monooleate, polysorbates, polaxomers, bile salts, glyceryl monostearate, copolymers of ethylene oxide and propylene oxide, e.g., Pluronic® (BASF), and the like. Some other surfactants include polyoxyethylene fatty acid glycerides and vegetable oils, e.g., polyoxyethylene (60) hydrogenated castor oil; and polyoxyethylene alkylethers and alkylphenyl ethers, e.g. octoxynol 10, octoxynol 40. In some embodiments, surfactants may be included to enhance physical stability or for other purposes.

In certain embodiments, the formulations may also include one or more viscosity enhancing agents which include, e.g., methyl cellulose, xanthan gum, carboxymethyl cellulose, hydroxypropyl cellulose, hydroxypropylmethyl cellulose, hydroxypropylmethyl cellulose acetate stearate, hydroxypropylmethyl cellulose phthalate, carbomer, polyvinyl alcohol alginates, acacia, chitosans and combinations thereof.

In some embodiments, the formulations may also include one or more wetting agents which include compounds such as oleic acid, glyceryl monostearate, sorbitan monooleate, sorbitan monolaurate, triethanolamine oleate, polyoxyethylene sorbitan monooleate, polyoxyethylene sorbitan monolaurate, sodium docusate, sodium oleate, sodium lauryl sulfate, sodium doccusate, triacetin, Tween 80, vitamin E TPGS, ammonium salts and the like.

It should be appreciated that there is considerable overlap between excipients used in the solid dosage forms described herein. Thus, the above-listed additives should be taken as merely exemplary, and not limiting, of the types of excipient that can be included in solid dosage forms described herein. The type and amounts of such excipient can be readily determined by one skilled in the art, according to the particular properties desired.

The present disclosure also includes the following embodiments.

In an embodiment, the solid oral dosage form is able to release less than about 10% by weight of Compound (I) and/or said pharmaceutically acceptable salt thereof in about 1.5 hours in a dissolution vessel comprising an aqueous solution at a pH of less than about 3; less than about 10% by weight of Compound (I) and/or said pharmaceutically acceptable salt thereof in about 1.5 hours in a dissolution vessel comprising an aqueous solution at a pH of from about 4.5 to about 5.0; and is able to release not less than about 80% by weight of Compound (I) and/or said pharmaceutically acceptable salt thereof from about twenty minutes to about two hours in a dissolution vessel comprising an aqueous solution at a pH of from about 6.4 to about 7.4. In this embodiment, typically the solid oral dosage form is able to release less than about 10% by weight of Compound (I) and/or said pharmaceutically acceptable salt thereof in about 1.5 hours in a dissolution vessel comprising an aqueous solution at a pH about 5.0. In this embodiment, typically the solid oral dosage form is able to release less than about 25% by weight of Compound (I) and/or said pharmaceutically acceptable salt thereof in about 15 minutes in a dissolution vessel comprising an aqueous solution at a pH of from about 6.4 to about 7.4. In this embodiment, typically the solid oral dosage form is able to release less than about 80% by weight of Compound (I) and/or said pharmaceutically acceptable salt thereof in about 30 minutes in a dissolution vessel comprising an aqueous solution at a pH of from about 6.4 to about 7.4. In this embodiment, typically the solid oral dosage form is able to release less than about 80% by weight of Compound (I) and/or said pharmaceutically acceptable salt thereof in about 45 minutes in a dissolution vessel comprising an aqueous solution at a pH of from about 6.4 to about 7.4. In this embodiment, typically the solid oral dosage form is able to release less than about 80% by weight of Compound (I) and/or said pharmaceutically acceptable salt thereof in about 60 minutes in a dissolution vessel comprising an aqueous solution at a pH of from about 6.4 to about 7.4. In this embodiment, typically the solid oral dosage form is able to release less than about 80% by weight of Compound (I) and/or said pharmaceutically acceptable salt thereof in about 120 minutes in a dissolution vessel comprising an aqueous solution at a pH of from about 6.4 to about 7.4.

In an embodiment, the aqueous solution is a simulated intestinal fluid at a pH of from about 6.4 to about 7.4.

In an embodiment, the solid oral dosage form comprises a pharmaceutically acceptable acid salt of Compound (I). Typically, the pharmaceutically acceptable acid salt is a sulfonic acid or carboxylic acid salt of Compound (I). For example, the acid may be mono- or di-methanesulfonic acid, mono or di-benezenesulfonic acid, mono- or di-toluenesulfonic acid, ethane-1,2-disulfonic acid, fumaric acid, oxalic acid, tartaric acid, maleic acid, citric acid, or malonic acid salt of Compound (I). For example, the acid may be mono- or di-methanesulfonic acid salt of Compound (I). Alternatively, the acid is citric acid salt of Compound (I).

In an embodiment, the solid oral dosage form comprises Compound (I).

In an embodiment, Compound (I) is an (E) and (Z) mixture of a mixture of (R) and (S) isomers of 2-[3-[4-amino-3-(2-fluoro-4-phenoxy-phenyl)-pyrazolo[3,4-d]pyrimidin-1-yl]piperidine-1-carbonyl]-4-methyl-4-[4-(oxetan-3-yl)piperazin-1-yl]pent-2-enenitrile.

In an embodiment, Compound (I) is an (E) and (Z) mixture of (R)-2-[3-[4-amino-3-(2-fluoro-4-phenoxy-phenyl)-pyrazolo[3,4-d]pyrimidin-1-yl]piperidine-1-carbonyl]-4-methyl-4-[4-(oxetan-3-yl)piperazin-1-yl]pent-2-enenitrile.

In an embodiment, at least about 85% by weight of Compound (I) and/or said pharmaceutically acceptable salt thereof is the (E) isomer.

In an embodiment, at least about 90% by weight of Compound (I) and/or said pharmaceutically acceptable salt thereof is the (E) isomer. The ratio of the E to Z isomer can be calculated by methods well known in the art. One such method is HPLC total area normalization method.

In an embodiment, Compound (I) and/or said pharmaceutically acceptable salt thereof is in substantially pure amorphous form.

In an embodiment, the solid oral dosage form further comprises an additional pharmaceutically acceptable acid in an amount sufficient to enhance dissolution of Compound (I) and/or said pharmaceutically acceptable salt thereof at a pH of from about 6.4 to about 7.4. Typically, the additional acid is an organic acid. For example, the additional acid may be succinic acid, citric acid, benzoic acid, tartaric acid, or malic acid. In this embodiment, the pharmaceutically acceptable acid typically forms an acidic aqueous solution within the solid oral dosage form prior to the release of Compound (I) and/or said pharmaceutically acceptable salt thereof, from the dosage form.

In an embodiment, the solid oral dosage form further comprises a surfactant present at a concentration above its critical micelle concentration upon disintegration in about 50 mL of aqueous media. In an alternative embodiment, the solid oral dosage form further comprises a surfactant present at a concentration above its critical micelle concentration upon disintegration in about 20 mL of aqueous media.

In an embodiment, the mean particle size of Compound (I) and/or said pharmaceutically acceptable salt thereof is from about 0.3 micron to about 100 microns. Typically, in this embodiment, the mean particle size of Compound (I) and/or said pharmaceutically acceptable salt thereof is from about 1 micron to about 50 microns. Alternatively, in this embodiment, the mean particle size of Compound (I) and/or said pharmaceutically acceptable salt thereof is less than or equal to about 15 micron.

In an embodiment, the enteric coating is coated with a topcoat. Typically, the topcoat is an immediate release coat.

In an embodiment, Compound (I) and/or said pharmaceutically acceptable salt thereof is admixed with a pharmaceutically acceptable excipient and/or layered on a particle, such as a seed, pellet, bead, etc. In this embodiment, the enteric coat is optionally coated with an immediate release topcoat and optionally with an intermediate seal coat, for example of low molecular weight hypromellose.

In an embodiment, the enteric coating is from about 10% to about 150% of the weight of the core material. Typically, in this embodiment, the enteric coating is from about 20% to about 100% of the weight of the core material. For example, the enteric coating may be from about 30% to about 60% of the weight of the core material. In this embodiment, the enteric coating may be from about 5 to about 500 microns thick. Typically, in this embodiment, the enteric coating is from about 8 to about 150 microns thick. For example, the enteric coating may be from about 50 to about 100 microns thick.

In an embodiment, the enteric coating is chosen from polymerized gelatin, shellac, methacrylic acid copolymer type CNF, cellulose butyrate phthalate, cellulose hydrogen phthalate, cellulose proprionate phthalate, polyvinyl acetate phthalate (PVAP), cellulose acetate phthalate (CAP), cellulose acetate trimellitate (CAT), hydroxypropyl methyl cellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and (meth)acrylic acid polymers and copolymers which polymers are made from one, and which copolymers are made from two or more monomers, chosen from methyl acrylate, ethyl acrylate, methyl methacrylate and ethyl methacrylate. Typically in this embodiment, the enteric coating is a poly(meth)acrylate polymer. Alternatively, the enteric coating is a Eudragit® L or S series. Alternatively, the enteric coating is a Eudragit® L100, L12.5, S100, S12.5, FS 30D, or a mixture of Eudragit L100 and S100.

In an embodiment, the enteric coating comprises a cellulose derivative. Typically, in this embodiment, the cellulose derivative is chosen from methylcellulose, cellulose acetate phthalate, hydroxymethyl cellulose phthalate (HPMCP), hydroxypropyl methyl cellulose succinate (HPMCS), and hydroxymethyl cellulose acetate succinate (HPMCAS).

In an embodiment, the enteric coating comprises a polyvinyl acetate phthalate (PVAP) polymer. Typically, in this embodiment, the subcoat is a water insoluble composition (such as a water insoluble polymer) and comprises particles of a water soluble compound capable of forming channels in the water insoluble composition and allowing influx of at least water into the solid oral dosage form and diffusion of Compound (I) and/or said pharmaceutically acceptable salt thereof into the intestine.

In an embodiment, the subcoat is a water insoluble composition (such as a water insoluble polymer) and comprises insoluble hydrophilic particles which causes swelling of said subcoat when in contact with an aqueous or gastric media.

In an embodiment, the subcoat comprises a water insoluble composition comprising particles of a water soluble compound capable of forming channels that is impermeable to Compound (I) and/or said pharmaceutically acceptable salt thereof, but allows entry of at least water and swelling and rupturing of the subcoat, causing release of Compound (I) and/or said pharmaceutically acceptable salt thereof, for example, as a bolus. For the purposes of this and other embodiments of this dosage form, bolus shall be interpreted as releasing at least about 80% of the contained Compound (I) and/or said pharmaceutically acceptable salt thereof in a period not more than about 1 hour in a dissolution testing under sink conditions at a pH of from about 6.4 to about 7.4.

In an embodiment, the solid oral dosage form is a tablet or a capsule.

In an embodiment, the pharmaceutically acceptable excipient is chosen from binders, surfactants, diluents, buffers, antiadherents, glidants, disintegrants, antioxidants, antifoaming agents, fillers, flavors, colors, lubricants, sorbents, preservatives, plasticizers, and sweeteners.

In an embodiment, the average systemic bioavailability of Compound (I) as measured by plasma AUC resulting from administration of said dosage form is from about 200% to 4000% of the average systemic bioavailability, as measured by plasma AUC, resulting from administration of an immediate release dosage form having an equivalent amount of Compound (I) and/or said pharmaceutically acceptable salt thereof.

In an embodiment, the average systemic bioavailability of Compound (I) as measured by plasma AUC resulting from administration of said pharmaceutical dosage form is from about 125% to about 2000% of the average systemic bioavailability as measured by plasma AUC resulting from administration of an intraduodenal release dosage form having an equivalent amount of Compound (I) and/or said pharmaceutically acceptable salt thereof.

The term "water insoluble hydrophilic particles" as used herein includes but is not limited to, polysaccharides including particles of calcium pectinate, calcium alginate, calcium xanthate, any metal salt of a polysaccharide containing an acid group where the salt renders the polysaccharide insoluble in water, microcrystalline starch, insoluble starch, any water insoluble polysaccharide (*e.g.,* cellulose or microcrystalline cellulose), any covalently crosslinked polysaccharide where said crosslinking renders the polysaccharide insoluble in water. Such crosslinking agents include, but are not limited to, glutaraldehyde, formaldehyde, epichlorohydrin, diacid chlorides, diisocyananates, diacid anhydrides and diamines.

The dosage forms disclosed herein can be obtained by mixing one or more solid excipient such as carrier, binder, filling agent, suspending agent, flavoring agent, sweetening agent, disintegrating agent, dispersing agent, surfactant, lubricant, colorant diluent, solubilizer, moistening agent, plasticizer, stabilizer, penetration enhancer, wetting agent, anti-foaming agent, antioxidant, preservative, or one or more combination thereof with Compound (I) or a pharmaceutically acceptable salt thereof, optionally grinding the resulting mixture, and processing the mixture of granules, after adding suitable excipients, if desired, to obtain tablets.

Pharmaceutical preparations disclosed herein also include capsules made of gelatin, as well as soft, sealed capsules made of gelatin and a plasticizer, such as glycerol or sorbitol. Capsules may also be made of polymers such as hypromellose. The capsules can contain the active ingredient in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, lipids, solubilizers, or liquid polyethylene glycols. In addition, stabilizers may be added. All formulations for oral administration should be in dosages suitable for such administration.

These formulations can be manufactured by conventional pharmacological techniques. Conventional pharmacological techniques include, e.g., one or a combination of methods: (1) dry mixing, (2) direct compression, (3) milling, (4) dry or non-aqueous granulation, (5) wet granulation, (6) fusion, or (7) extrusion. *See,* e.g., Lachman et al., The Theory and Practice of Industrial Pharmacy, 3rd ed. (1986). Other methods include, e.g., spray drying, pan coating, melt granulation, granulation, fluidized bed spray drying or coating (e.g., Wurster coating), tangential coating, top spraying, tableting, extruding, extrusion/ spheronization, and the like.

The enteric coated tablets and capsules dosage forms containing the disclosed compound can be made by methods well known in the art. For example, tablets containing a compound disclosed herein can be enterically coated with a coating solution containing a Eudragit®, diethylphthlate, isopropyl alcohol, talc, and water using a side vented coating pan (Freund Hi-Coater).

Alternatively, a multi-unit dosage form comprising enteric-coated pellets comprising Compound (I) that can be incorporated into a tablet or into a capsule can be prepared as follows.

### Core material:

The core material for the individually enteric coating layered pellets can be constituted according to different principles. Seeds (*e.g.*, SODAS®) layered with the active agent, optionally mixed with alkaline substances or buffer, can be used as the core material for the further processing.

The seeds which are to be layered with the active agent can be water insoluble seeds comprising different oxides, celluloses, organic polymers and other materials, alone or in mixtures or water-soluble seeds comprising different inorganic salts, sugars, non-pareils and other materials, alone or in mixtures. Further, the seeds may comprise the active agent in the form of crystals, agglomerates, compacts etc. The size of the seeds is not essential for the present invention but may vary between approximately 0.1 and 2 mm. The seeds layered with the active agent are produced either by powder or solution/suspension layering using for instance granulation or spray coating layering equipment. The seeds are covered by a subcoat and an enteric coating.

Before the seeds are layered, the active agent may be mixed with a suitble excipient such as binder, surfactant, filler, disintegrating agent, alkaline additive, and the like. The binders are for example polymers such as hydroxypropyl methylcellulose (HPMC), hydroxypropyl-cellulose (HPC), carboxymethylcellulose sodium, polyvinyl pyrrolidone (PVP), or sugars, starches or other pharmaceutically acceptable substances with cohesive properties. Suitable surfactants are found in the groups of pharmaceutically acceptable nonionic or ionic surfactants such as for instance sodium lauryl sulfate.

Alternatively, the active agent optionally mixed with suitable excipients can be formulated into a core material. Said core material may be produced by extrusion/ spheronization, balling or compression utilizing conventional process equipment. The size of the formulated core material is approximately between 0.1 and 4 mm and for example, between 0.1 and 2 mm. The manufactured core material can further be layered with additional excipients comprising the active agent and/or be used for further processing.

The active agent is mixed with pharmaceutical excipients to obtain preferred handling and processing properties and a suitable concentration of the active agent in the final preparation. Pharmaceutical excipients such as fillers, binders, lubricants, disintegrating agents, surfactants and other pharmaceutically acceptable additives may be used.

Alternatively, the aforementioned core material can be prepared by using spray drying or spray congealing technique.

### Enteric Coating Layer(s):

Before applying the enteric coating layer(s) onto the core material in the form of individual pellets, the pellets are covered with a subcoat and may also optionally be covered with one or more separating layer(s) comprising pharmaceutical excipients optionally including alkaline compounds such as pH-buffering compounds. This/these separating layer(s), separate(s) the core material from the outer layers being enteric coating layer(s). This/these separating layer(s) protecting the core material of active agent should be water soluble or rapidly disintegrating in water.

The subcoat: (1) is a water soluble or hydrophilic erodible polymer, said polymer being a low molecular weight polymer selected from hydroxymethyl cellulose (HPMC), hydroxyethyl cellulose, hydroxypropyl cellulose, microcrystalline cellulose, polyvinylpyrrolidones, polysaccharides, a polysaccharide derivative, polyvinyl alcohols, polyethylene glycol (PEG), polypropylene glycol (PPG), and a PEG-PPG block copolymer; or (2) comprises a water insoluble composition comprising: (i) particles of a water soluble compound capable of forming channels in the water insoluble composition; or (ii) water insoluble hydrophilic particles which cause swelling of the subcoat when in contact with an aqueous media.

A separating layer(s) can be optionally applied to the core material by coating or layering procedures in suitable equipments such as coating pan, coating granulator or in fluidized bed apparatus using water and/or organic solvents for the coating process. As an alternative the separating layer(s) can be applied to the core material by using powder coating technique. The materials for the separating layers are pharmaceutically acceptable compounds such as, for instance, sugar, polyethylene glycol, polyvinylpyrrolidone, polyvinyl alcohol, polyvinyl acetate, hydroxypropyl cellulose, methylcellulose, ethylcellulose, hydroxypropyl methyl cellulose, carboxymethylcellulose sodium, water soluble salts of enteric coating polymers and others, used alone or in mixtures. Additives such as plasticizers, colorants, pigments, fillers anti-tacking and anti-static agents, such as for instance magnesium stearate, titanium dioxide, talc and other additives may also be included into the separating layer(s).

When the optional separating layer is applied to the core material, it may constitute a variable thickness. The maximum thickness of the separating layer(s) is normally only limited by processing conditions. The separating layer may serve as a diffusion barrier and may act as a pH-buffering zone. The optionally applied separating layer(s) is not essential for the invention. However, the separating layer(s) may improve the chemical stability of the active substance and/or the physical properties of the novel multiple unit tableted dosage form.

Alternatively, the separating layer may be formed *in situ* by a reaction between an enteric coating polymer layer applied on the core material and an alkaline reacting compound in the core material. Thus, the separating layer formed comprises a water soluble salt formed between the enteric coating layer polymer(s) and an alkaline reacting compound which is in the position to form a salt.

One or more enteric coating layers are applied by using a suitable coating technique. The enteric coating layer material may be dispersed or dissolved in either water or in suitable organic solvents. As enteric coating layer polymers one or more, separately or in combination, of the following can be used, e.g. solutions or dispersions of methacrylic acid copolymers, cellulose acetate phthalate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating polymer(s).

The enteric coating layers contain pharmaceutically acceptable plasticizers to obtain the desired mechanical properties, such as flexibility and hardness of the enteric coating layers. Such plasticizers are for instance, but not restricted to triacetin, citric acid esters, phthalic acid esters, dibutyl sebacate, cetyl alcohol, polyethylene glycols, polysorbates or other plasticizers.

The amount of plasticizer is optimized for each enteric coating layer formula, in relation to the selected enteric coating layer polymer(s), selected plasticizer(s) and the applied amount of said polymer(s), in such a way that the mechanical properties, i.e. flexibility and hardness of the enteric coating layer(s), for instance exemplified as Vickers hardness, are adjusted so that if a tablet is desired the acid resistance of the pellets covered with enteric coating layer(s) does not decrease significantly during compression of pellets into tablets. The amount of plasticizer is usually above 5% by weight of the enteric coating layer polymer(s), such as 15-50% and further such as 20-50%. Additives such as dispersants, colorants, pigments polymers e.g. poly(ethylacrylate, methylmethacrylate), anti-tacking and anti-foaming agents may also be included into the enteric coating layer(s). Other compounds may be added to increase film thickness and to decrease diffusion of acidic gastric juices into the acid susceptible material. The maximum thickness of the applied enteric coating is normally only limited by processing conditions and the desired dissolution profile.

### Over-Coating Layer:

Pellets covered with enteric coating layer(s) may optionally further be covered with one or more over-coating layer(s). The over-coating layer(s) should be water soluble or rapidly disintegrating in water. The over-coating layer(s) can be applied to the enteric coating layered pellets by coating or layering procedures in suitable equipments such as coating pan, coating granulator or in a fluidized bed apparatus using water and/or organic solvents for the coating or layering process. The materials for over-coating layers are chosen among pharmaceutically acceptable compounds such as, for instance sugar, polyethylene glycol, polyvinylpyrrolidone, polyvinyl alcohol, polyvinyl acetate, hydroxypropyl cellulose, methylcellulose, ethylcellulose, hydroxypropyl methyl cellulose, carboxymethylcellulose sodium and others, used alone or in mixtures. Additives such as plasticizers, colorants, pigments, fillers, anti-tacking and anti-static agents, such for instance magnesium stearate, titanium dioxide, talc and other additives may also be included into the over-coating layer(s). The over-coating layer may further prevent potential agglomeration of enteric coating layered pellets, further it may protect the enteric coating layer towards cracking during the compaction process and enhance the tableting process. The maximum thickness of the applied over-coating layer(s) is normally limited by processing conditions and the desired dissolution profile. The over-coating layer may also be used as a tablet film coating layer.

Enteric coating of soft gelatin capsules may contain an emulsion, oil, microemulsion, self-emulsifying system, lipid, triglycerides, polyethylene glycol, surfactants, other solubilizers and the like, and combinations thereof, to solubilize the active agent. The flexibility of the soft gelatin capsule is maintained by residual water and plasticizer. Moreover, for gelatin capsules the gelatin may be dissolved in water so that spraying must be accomplished at a rate with relatively low relative humidity such as can be accomplished in a fluid bed or Wurster. In addition, drying should be accomplished without removing the residual water or plasticizer causing cracking of the capsule shell. Commercially available blends optimized for enteric coating of soft gelatin capsules such as Instamodel EPD (Enteric Polymeric Dispersion), available from Ideal Cures, Pvt. Ltd. (Mumbai, India). On a laboratory scale enteric coated capsules may be prepared by: a) rotating capsules in a flask or dipping capsules in a solution of the gently heated enteric coating material with plasticizer at the lowest possible temperature or b) in a lab scale sprayer/fluid bed and then drying.

For aqueous active agents, it can be especially desirable to incorporate the drug in the water phase of an emulsion. Such "water-in-oil" emulsion provides a suitable biophysical environment for the drug and can provide an oil-water interface that can protect the drug from adverse effects of pH or enzymes that can degrade the drug. Additionally, such water-in-oil formulations can provide a lipid layer, which can interact favorably with lipids in cells of the body, and can increase the partition of the formulation onto the membranes of cells. Such partition can increase the absorption of drugs in such formulations into the circulation and therefore can increase the bioavailability of the drug. The aqueous phase may optionally comprise the active agent suspended in water and a buffer.

In some embodiments the water-in-oil emulsion contains an oily phase composed of long chain carboxylic acids or esters or alcohols thereof, a surfactant or a surface active agent, and an aqueous phase containing primarily water and the active agent.

Long chain carboxylic acids are those ranging from C₈ to C₂₂ with up to three unsaturated bonds (also branching). Examples of saturated straight chain acids are n-dodecanoic acid, n-tetradecanoic acid, n-hexadecanoic acid, caproic acid, caprylic acid, capric acid, lauric acid, myristic acid, palmitic acid, stearic acid, arachidic acid, behenic acid, montanic acid and melissic acid. Also useful are unsaturated monoolefinic straight chain monocarboxylic acids. Examples of these are oleic acid, gadoleic acid and erucic acid. Also useful are unsaturated (polyolefinic) straight chain monocarboxylic acids. Examples of these are linoleic acid, ricinoleic acid, linolenic acid, arachidonic acid and behenolic acid. Useful branched acids include, for example, diacetyl tartaric acid.

Examples of long chain carboxylic acid esters include, but are not limited to, those from the group of: glyceryl monostearates; glyceryl monopalmitates; mixtures of glyceryl monostearate and glyceryl monopalmitate; glyceryl monolinoleate; glyceryl monooleate; mixtures of glyceryl monopalmitate, glyceryl monostearate, glyceryl monooleate and glyceryl monolinoleate; glyceryl monolinolenate; glyceryl monogadoleate; mixtures of glyceryl monopalmitate, glyceryl monostearate, glyceryl monooleate, glyceryl monolinoleate, glyceryl monolinolenate and glyceryl monogadoleate; acetylated glycerides such as distilled acetylated monoglycerides; mixtures of propylene glycol monoesters, distilled monoglycerides, sodium steroyl lactylate and silicon dioxide; d-alpha tocopherol polyethylene glycol 1000 succinate; mixtures of mono- and di-glyceride esters such as Atmul; calcium stearoyl lactylate; ethoxylated mono- and di-glycerides; lactated mono- and di-glycerides; lactylate carboxylic acid ester of glycerol and propylene glycol; lactylic esters of long chain carboxylic acids; polyglycerol esters of long chain carboxylic acids, propylene glycol mono- and di-esters of long chain carboxylic acids; sodium stearoyl lactylate; sorbitan monostearate; sorbitan monooleate; other sorbitan esters of long chain carboxylic acids; succinylated monoglycerides; stearyl monoglyceryl citrate; stearyl heptanoate; cetyl esters of waxes; stearyl octanoate; C₈-C₃₀ cholesterol/lavosterol esters; and sucrose long chain carboxylic acid esters. Examples of the self-emulsifying long chain carboxylic acid esters include those from the groups of stearates, pamitates, ricinoleates, oleates, behenates, ricinolenates, myristates, laurates, caprylates, and caproates. In some embodiments the oily phase may comprise a combination of 2 or more of the long chain carboxylic acids or esters or alcohols thereof. In some embodiments the oil phase may comprise a mixture of caprylic/capric triglyceride and C₈/C ₁₀ mono-/di-glycerides of caprylic acid.

The alcohols that can be used are exemplified by the hydroxyl forms of the carboxylic acids exemplified above and also strearyl alcohol.

Surface active agents or surfactants are long chain molecules that can accumulate at hydrophilic/hydrophobic (water/oil) interfaces and lower the surface tension at the interface. As a result they can stabilise an emulsion. In some embodiments of this invention, the surfactant may comprise: Tween® (polyoxyethylene sorbate) family of surfactants, Span® (sorbitan long chain carboxylic acid esters) family of surfactants, Pluronic® (ethylene or propylene oxide block copolymers) family of surfactants, Labrasol®, Labrafil® and Labrafac®(each polyglycolyzed glycerides) families of surfactants, sorbitan esters of oleate, stearate, laurate or other long chain carboxylic acids, poloxamers (polyethylene-polypropylene glycol block copolymers or Pluronic®), other sorbitan or sucrose long chain carboxylic acid esters, mono and diglycerides, PEG derivatives of caprylic/capric triglycerides and mixtures thereof or mixture of two or more of the above. In some embodiments the surfactant phase may comprise a mixture of Polyoxyethylene (20) sorbitan monooleate (Tween 80®) and sorbitan monooleate (Span 80®). The aqueous phase may optionally comprise the active agent suspended in water and a buffer.

In some embodiments, such emulsions are coarse emulsions, microemulsions and liquid crystal emulsions. In other embodiments such emulsion may optionally comprise a permeation enhancer. In other embodiments, spray-dried dispersions or microparticles or nanoparticles containing encapsulated microemulsion, coarse emulsion or liquid crystal can be used.

In some embodiments, the solid dosage forms described herein are non-enteric time-delayed release dosage forms. The term "non-enteric time-delayed release" as used herein refers to the delivery so that the release of the drug can be accomplished at some generally predictable location in the intestinal tract more distal to that which would have been accomplished if there had been no delayed release alterations. In some embodiments the method for delay of release is a coating that becomes permeable, dissolves, ruptures, and/or is no longer intact after a designed duration.

The coating in the time-delayed release dosage forms can have a fixed time to erode after which the drug is released (suitable coating include polymeric coating such as HPMC, and the like) or has a core comprised of a superdisinegrant(s) or osmotic agent(s) such as a salt, hydrophilic polymer, typically polyethylene oxide or an alkylcellulose, sugar, or the like, which draw water through a membrane or a gas generating agent such as citric acid and sodium bicarbonate. The membrane may rupture after the swelling pressure exceeds a certain threshold over a desired delay time. Alternatively, a membrane could become porous by leaching an aqueous extractable containing the drug over a desired delay time. The time delayed dosage forms are for example administered in a fasted state to avoid the variability in gastric emptying in the fed state.

The time- delayed dosage form can be a mechanical pill such as an Enterion® capsule or Heidelberg® capsule (pH sensitive) which can release the drug when it receives a signal which can be transmitted once it leaves the stomach.

In the dosage form either the enteric coating or a subcoating can used to delay release of the drug in the regions of interest. In the dosage form where the enteric coating provides for the release of the drug in duodenum, jejunum, ileum, or colon, preferably jejunum or ileum, the enteric coating dissolves more slowly so that the dosage form can transit further down the intestine to the area of intended drug release. Examples of enteric coatings that can be used to targeted release of the drug in the small intestine may be formulated using several approaches. One approach is by combining different kinds of Eudragit with a small percentage of an Eudragit that dissolves at a higher pH to slow down the dissolution, for example, an Eudragit that dissolves at pH 5.5 may be blended with a small percentage (97:3 to 5:1 w/w) Eudragit that dissolves at pH 6 or pH 7. Alternatively, a small amount of acid, such as citric acid or fumaric acid, may be included in the coating to lower the pH. Another approach is to add less soluble polymers to the enteric coating to retard dissolution. In addition, the level of plasticizers, pore formers, or the thickness of the coating can be used to alter the rate of dissolution of the enteric coating.

When the subcoating provides the delay, the enteric coating is designed to erode faster, for example, within 10 to 30 minutes. Examples of enteric coatings that dissolve in the duodenum are 1) Eudragit L30-D-55 and L100-55 that dissolve above pH 5.5 and 2) Eudragit L100 and L12,5 that dissolve at pH 6.0. The subcoating such as erosional coatings, osmotic pumps, or burstable osmotic pumps then causes release of the drug in the regions of interest. The subcoat has a fixed time to erode after which the drug is released (suitable coating include polymeric coating such as HPMC, and the like) or has a core comprised of a superdisinegrant(s) or osmotic agent(s) such as a salt, hydrophilic polymer, typically polyethylene oxide or an alkylcellulose, sugar, or the like, which draw water through a membrane or a gas generating agent such as citric acid and sodium bicarbonate. The membrane may rupture after the swelling pressure exceeds a certain threshold over a desired delay time. Alternatively, a membrane could become porous by leaching an aqueous extractable over a desired delay time.

### Administration

In general, the compounds of this disclosure will be administered in a therapeutically effective amount. Therapeutically effective amounts of the compounds disclosed herein may range from about 300 to about a gram. The actual amount administered of the compound of this disclosure, i.e., the sulfonic acid salt of Compound (I), carboxylic acid salt of Compound (I) or an amorphous form of a pharmaceutically acceptable salt of Compound (I) and any embodiments thereof disclosed above, will depend upon numerous factors such as the severity of the disease to be treated, the age and relative health of the mammal, the potency of the compound and/or pharmaceutically acceptable salt thereof being utilized, the route and form of administration, and other factors.

The compounds of the present disclosure may be used in combination with one or more other drugs in the treatment of diseases or conditions for which compounds of the present disclosure or the other drugs may have utility, where the combination of the drugs together are safer or more effective than either drug alone. Such other drug(s) may be administered, by a route and in an amount commonly used therefore, contemporaneously or sequentially with a compound of the present disclosure. When a compound of the present disclosure is used contemporaneously with one or more other drugs, a pharmaceutical composition in unit dosage form containing such other drugs and the compound of the present disclosure is preferred. However, the combination therapy may also include therapies in which the compound of the present disclosure and one or more other drugs are administered on different overlapping schedules. It is also contemplated that when used in combination with one or more other active ingredients, the compounds of the present disclosure and the other active ingredients may be used in lower doses than when each is used singly.

Accordingly, the pharmaceutical compositions of the present disclosure also include those that contain one or more other active ingredients, in addition to a compound of the present disclosure.

The above combinations include combinations of a compound of the present disclosure not only with one other active compound, but also with two or more other active compounds. Likewise, compounds of the present disclosure may be used in combination with other drugs that are used in the prevention, treatment, control, amelioration, or reduction of risk of the diseases or conditions for which compounds of the present disclosure are useful. Such other drugs may be administered, by a route and in an amount commonly used therefore by those skilled in the art, contemporaneously or sequentially with a compound of the present disclosure. When a compound of the present disclosure is used contemporaneously with one or more other drugs, a pharmaceutical composition containing such other drugs in addition to the compound of the present disclosure is preferred. Accordingly, the pharmaceutical compositions of the present disclosure also include those that also contain one or more other active ingredients, in addition to a compound of the present disclosure. The weight ratio of the compound of the present disclosure to the second active ingredient may be varied and will depend upon the effective dose of each ingredient. Generally, an effective dose of each will be used.

Where the mammal is suffering from or at risk of suffering from an autoimmune disease, an inflammatory disease, or an allergy disease, a compound of present disclosure can be used in with one or more of the following therapeutic agents in any combination: immunosuppressants (e.g., tacrolimus, - 38 -iethylstilb, rapamicin, methotrexate, cyclophosphamide, azathioprine, mercaptopurine, mycophenolate, or FTY720), glucocorticoids (e.g., prednisone, cortisone acetate, prednisolone, methylprednisolone, dexamethasone, betamethasone, triamcinolone, beclometasone, fludrocortisone acetate, deoxycorticosterone acetate, aldosterone), non-steroidal anti-inflammatory drugs (e.g., salicylates, arylalkanoic acids, 2-arylpropionic acids, N-arylanthranilic acids, oxicams, coxibs, or sulphonanilides), Cox-2-specific inhibitors (e.g., valdecoxib, celecoxib, or rofecoxib), leflunomide, gold thioglucose, gold thiomalate, aurofin, sulfasalazine, hydroxychloroquinine, minocycline, TNF-.alpha. binding proteins (e.g., infliximab, etanercept, or adalimumab), abatacept, anakinra, interferon-.beta., interferon-.gamma., interleukin-2, allergy vaccines, antihistamines, antileukotrienes, beta-agonists, theophylline, and anticholinergics.

Where the mammal is suffering from or at risk of suffering from a B-cell proliferative disorder (e.g., plasma cell myeloma), the mammalcan be treated with a compound disclosed herein in any combination with one or more other anti-cancer agents. In some embodiments, one or more of the anti-cancer agents are proapoptotic agents. Examples of anti-cancer agents include, but are not limited to, any of the following: gossyphol, genasense, polyphenol E, Chlorofusin, all trans-retinoic acid (ATRA), bryostatin, tumor necrosis factor-related apoptosis-inducing ligand (TRAIL), 5-aza-2'-deoxycytidine, all trans retinoic acid, doxorubicin, vincristine, etoposide, gemcitabine, imatinib (Gleevec™), geldanamycin, 17-N-Allylamino-17-Demethoxygeldanamycin (17-AAG), flavopiridol, LY294002, bortezomib, trastuzumab, BAY 11-7082, PKC412, or PD184352, Taxol™, also referred to as "paclitaxel", which is a well-known anti-cancer drug which acts by enhancing and stabilizing microtubule formation, and docetaxol, such as Taxotere™. Compounds that have the basic taxane skeleton as a common structure feature, have also been shown to have the ability to arrest cells in the G2-M phases due to stabilized microtubules and may be useful for treating cancer in combination with the compounds described herein.

Further examples of anti-cancer agents for use in combination with a compound disclosed herein include inhibitors of mitogen-activated protein kinase signaling, e.g., U0126, PD98059, PD184352, PD0325901, ARRY-142886, SB239063, SP600125, BAY 43-9006, wortmannin, or LY294002; Syk inhibitors; mTOR inhibitors; and antibodies (e.g., rituxan).

Other anti-cancer agents that can be employed in combination with a compound disclosed herein include Adriamycin, Dactinomycin, Bleomycin, Vinblastine, Cisplatin, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cladribine; crisnatol mesylate; cyclophosphamide; cytarabine; dacarbazine; daunorubicin hydrochloride; decitabine; dexormaplatin; dezaguanine; dezaguanine mesylate; diaziquone; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-nl; interferon alfa-n3; interferon beta-la; interferon gamma-1 b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other anti-cancer agents that can be employed in combination with a compound and/or pharmaceutically acceptable salt disclosed herein include: 20-epi-1, 25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; 9-dioxamycin; diphenyl spiromustine; docosanol; dolasetron; doxifluridine; droloxifene; dronabinol; duocarmycin SA; ebselen; ecomustine; edelfosine; edrecolomab; eflomithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; fmasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+- 42 -iethylstilbe cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylerie conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; R.sub.11 retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived 1; sense oligonucleotides; signal transduction inhibitors; signal transduction modulators; single chain antigen-binding protein; sizofuran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

Yet other anticancer agents that can be employed in combination with a compound disclosed herein include alkylating agents, antimetabolites, natural products, or hormones, e.g., nitrogen mustards (e.g., mechloroethamine, cyclophosphamide, chlorambucil, etc.), alkyl sulfonates (e.g., busulfan), nitrosoureas (e.g., carmustine, lomusitne, etc.), or triazenes (decarbazine, etc.). Examples of antimetabolites include but are not limited to folic acid analog (e.g., methotrexate), or pyrimidine analogs (e.g., Cytarabine), purine analogs (e.g., mercaptopurine, thioguanine, pentostatin).

Examples of natural products useful in combination with a compound disclosed herein include but are not limited to vinca alkaloids (e.g., - 43 -iethylstil, vincristine), epipodophyllotoxins (e.g., etoposide), antibiotics (e.g., daunorubicin, doxorubicin, bleomycin), enzymes (e.g., L-asparaginase), or biological response modifiers (e.g., interferon alpha).

Examples of alkylating agents that can be employed in combination a compound disclosed herein include, but are not limited to, nitrogen mustards (e.g., mechloroethamine, cyclophosphamide, chlorambucil, melphalan, etc.), ethylenimine and methylmelamines (e.g., hexamethlymelamine, thiotepa), alkyl sulfonates (e.g., busulfan), nitrosoureas (e.g., carmustine, lomusitne, semustine, streptozocin, etc.), or triazenes (decarbazine, etc.). Examples of antimetabolites include, but are not limited to folic acid analog (e.g., methotrexate), or pyrimidine analogs (e.g., fluorouracil, floxuridine, Cytarabine), purine analogs (e.g., mercaptopurine, thioguanine, pentostatin.

Examples of hormones and antagonists useful in combination with a compound disclosed herein include, but are not limited to, adrenocorticosteroids (e.g., prednisone), progestins (e.g., hydroxyprogesterone caproate, megestrol acetate, medroxyprogesterone acetate), estrogens (e.g., diethylstilbestrol, ethinyl estradiol), antiestrogen (e.g., tamoxifen), androgens (e.g., testosterone propionate, fluoxymesterone), antiandrogen (e.g., flutamide), gonadotropin releasing hormone analog (e.g., leuprolide). Other agents that can be used in the methods and compositions described herein for the treatment or prevention of cancer include platinum coordination complexes (e.g., cisplatin, carboblatin), anthracenedione (e.g., mitoxantrone), substituted urea (e.g., hydroxyurea), methyl hydrazine derivative (e.g., procarbazine), adrenocortical suppressant (e.g., mitotane, aminoglutethimide).

Examples of anti-cancer agents which act by arresting cells in the G2-M phases due to stabilized microtubules and which can be used in combination with a compound of the present disclosure include without limitation the following marketed drugs and drugs in development: Erbulozole (also known as R-55104), Dolastatin 10 (also known as DLS-10 and NSC-376128), Mivobulin isethionate (also known as CI-980), Vincristine, NSC-639829, Discodermolide (also known as NVP-XX-A-296), ABT-751 (Abbott, also known as E-7010), Altorhyrtins (such as Altorhyrtin A and Altorhyrtin C), Spongistatins (such as Spongistatin 1, Spongistatin 2, Spongistatin 3, Spongistatin 4, Spongistatin 5, Spongistatin 6, Spongistatin 7, Spongistatin 8, and Spongistatin 9), Cemadotin hydrochloride (also known as LU-103793 and NSC-D-669356), Epothilones (such as Epothilone A, Epothilone B, Epothilone C (also known as desoxyepothilone A or dEpoA), Epothilone D (also referred to as KOS-862, dEpoB, and desoxyepothilone B), Epothilone E, Epothilone F, Epothilone B N-oxide, Epothilone A N-oxide, 16-aza-epothilone B, 21-aminoepothilone B (also known as BMS-310705), 21-hydroxyepothilone D (also known as Desoxyepothilone F and dEpoF), 26-fluoroepothilone), Auristatin PE (also known as NSC-654663), Soblidotin (also known as TZT-1027), LS-4559-P (Pharmacia, also known as LS-4577), LS-4578 (Pharmacia, also known as LS-477-P), LS-4477 (Pharmacia), LS-4559 (Pharmacia), RPR-112378 (Aventis), Vincristine sulfate, DZ-3358 (Daiichi), FR-182877 (Fujisawa, also known as WS-9885B), GS-164 (Takeda), GS-198 (Takeda), KAR-2 (Hungarian Academy of Sciences), BSF-223651 (BASF, also known as ILX-651 and LU-223651), SAH-49960 (Lilly/Novartis), SDZ-268970 (Lilly/Novartis), AM-97 (Armad/Kyowa Hakko), AM-132 (Armad), AM-138 (Armad/Kyowa Hakko), IDN-5005 (Indena), Cryptophycin 52 (also known as LY-355703), AC-7739 (Ajinomoto, also known as AVE-8063A and CS-39.HCl), AC-7700 (Ajinomoto, also known as AVE-8062, AVE-8062A, CS-39-L-Ser.HCl, and RPR-258062A), Vitilevuamide, Tubulysin A, Canadensol, Centaureidin (also known as NSC-106969), T-138067 (Tularik, also known as T-67, TL-138067 and TI-138067), COBRA-1 (Parker Hughes Institute, also known as DDE-261 and WHI-261), H10 (Kansas State University), H16 (Kansas State University), Oncocidin A1 (also known as BTO-956 and DIME), DDE-313 (Parker Hughes Institute), Fijianolide B. Laulimalide, SPA-2 (Parker Hughes Institute), SPA-1 (Parker Hughes Institute, also known as SPIKET-P), 3-IAABU (Cytoskeleton/Mt. Sinai School of Medicine, also known as MF-569), Narcosine (also known as NSC-5366), Nascapine, D-24851 (Asta Medica), A-105972 (Abbott), Hemiasterlin, 3-BAABU (Cytoskeleton/Mt. Sinai School of Medicine, also known as MF-191), TMPN (Arizona State University), Vanadocene acetylacetonate, T-138026 (Tularik), Monsatrol, Inanocine (also known as NSC-698666), 3-1AABE (Cytoskeleton/Mt. Sinai School of Medicine), A-204197 (Abbott), T-607 (Tuiarik, also known as T-900607), RPR-115781 (Aventis), Eleutherobins (such as Desmethyleleutherobin, Desaetyleleutherobin, Isoeleutherobin A, and Z-Eleutherobin), Caribaeoside, Caribaeolin, Halichondrin B, D-64131 (Asta Medica), D-68144 (Asta Medica), Diazonamide A, A-293620 (Abbott), NPI-2350 (Nereus), Taccalonolide A, TUB-245 (Aventis), A-259754 (Abbott), Diozostatin, (-)-Phenylahistin (also known as NSCL-96F037), D-68838 (Asta Medica), D-68836 (Asta Medica), Myoseverin B, D-43411 (Zentaris, also known as D-81862), A-289099 (Abbott), A-318315 (Abbott), HTI-286 (also known as SPA-110, trifluoroacetate salt) (Wyeth), D-82317 (Zentaris), D-82318 (Zentaris), SC-12983 (NCI), Resverastatin phosphate sodium, BPR-OY-007 (National Health Research Institutes), and SSR-250411 (Sanofi).

Where the mammal is suffering from or at risk of suffering from a thromboembolic disorder (e.g., stroke), the mammal can be treated with a compound disclosed herein in any combination with one or more other anti-thromboembolic agents. Examples of anti-thromboembolic agents include, but are not limited to, any of the following: thrombolytic agents (e.g., alteplase anistreplase, streptokinase, urokinase, or tissue plasminogen activator), heparin, tinzaparin, warfarin, dabigatran (e.g., dabigatran etexilate), factor Xa inhibitors (e.g., fondaparinux, draparinux, rivaroxaban, DX-9065a, otamixaban, LY517717, or YM150), ticlopidine, clopidogrel, CS-747 (prasugrel, LY640315), ximelagatran, or BIBR 1048.

### EXAMPLES

The following examples illustrate the preparation of compositions within the scope of the invention. These compositions are provided to enable those skilled in the art to more clearly understand and to practice the present invention. They should not be considered as limiting the scope of the invention, but merely as being illustrative and representative thereof.

### Examples

### Methods of Analysis

¹H-NMR experiments were performed on a Bruker AV400 (¹H frequency: 400 MHz). ¹H-NMR experiments of each sample were performed in DMSO-d₆ or CDCl₃ and each sample was prepared to *ca.* 5mg/mL concentration.

Ion chromatography was conducted on Dioned ICS-3000 ion chromatograph equipmed with Dionex Ionpac AS11-HC, 4 x 250 mm column with AG11-HC colum guard at 1.5 ml/min at 30 °C. The eluent was 5 mM NaOH. Ions were detected using a conductivity detector.

The XRPD analysis was carried out on a Siemens D5000 diffractometer, scanning the samples between 3 and 30 °2-theta (between 3 and 50 °2-theta when analysing input materials) with Cu K-alpha radiation source. The material was gently compressed onto a glass disc inserted into an XRPD sample holder. The samples were then loaded into the diffractometer running in reflection mode and analysed.

### Example 1

### Determination of E and Z isomer ratio in a (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile

High Performance Liquid Chromatograophy (HPLC) was conducted on Agilent 1100 equipped with a column heater, gradient elution capability, an autosampler and a UV detector. The column was Zorbax SB-Phenyl at 40 °C and the eluent was water/methanol gradient with 0.1% methane sulfonic acid and UV detection at 225nm. Total run time was 8 minutes. The following gradient was used (A is water, and B is methanol):

| Minutes | %A | %B |
|---|---|---|
| 0.0 | 40 | 60 |
| 5.0 | 20 | 80 |
| 7.0 | 20 | 80 |
| 7.25 | 40 | 60 |
| 8.0 | 40 | 60 |

### Salt formation

### Example 2

### Preparation of hemisulfate and sulfate salt of (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitiile having about 9/1 E/Z ratio

### Hemisulfate:

To the solution of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (having about 9/1 E/Z ratio) (4.2 g) in EtOAc (60 mL, 15 vol) was added sulfuric acid (0.31 g, 0.17 mL, 0.5 eq) in EtOAc (20 mL, 5 vol) at ambient temperature. The suspension was stirred at ambient temperature for ∼ 2 hr and then 40 °C for 4 hr and then at ambient temperature for at least 1 hr. After filtration and drying at ambient temperature under vacuum, 1.5 g of white powder was obtained. Solubility of the hemi-sulfate at ambient temperature was > 100 mg/mL in water.

### Sulfate salt

To the solution of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (having about 9/1 E/Z ratio) (810 mg) in EtOAc (8 mL, 10 vol) was added sulfuric acid (0.06 mL, 1.0 equiv.) in EtOAc (2.5 mL, 5 vol) at ambient temperature. The resulting suspension was stirred at 40 °C for 2 hr and then cooled to ambient temperature for at least 1 hr. After filtration, solids were dried by suction under Argon for 1 h to give a white powder (0.68 g) in 69% yield.

| Salt form | Solvent | XRD |
|---|---|---|
| H₂SO₄ | EtOAc | Amorphous |
| 0.5 H₂SO₄ | EtOAc | Amorphous |

### Example 3

### Preparation of hydrochloride salt of (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitiile having about 9/1 E/Z ratio

To a solution of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (having about 9/1 E/Z ratio) (100 mg, 0.15 mmol) in CH₂Cl₂ (1ml) at ambient temperature was added 2 equivalent of HCl (0.3 mmol, 0.15 ml of 2M HCl in 1:1 dioaxane:CH₂Cl₂). The resulting homogeneous solution was stirred at ambient temperature for 1 h and was added dropwise to 15 volumes of ethylacetate (as compared to CH₂Cl₂) resulting in formation of a white solid. The mixtures was aged at ambient temperature for 1h and placed at 2-8 C for 19 h. Upon filtration and washing of the filter cake with ethylacetate and drying a white solid was obtained. Analysis by XRPD indicated formation of an amorphous solid. Both ¹H-NMR and IC analysis indicated formation of the salt. IC indicated formation mono-HCl salt.

| Salt form | Solvent | Anti solvent | XRPD |
|---|---|---|---|
| HCl | CH₂Cl₂ | EtOAc | Amorphous |

### Example 4

### General procedure for preparation of mono- and di-mesylate salts of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile having about 9/1 E/Z ratio

To a solution of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (having about 9/1 E/Z ratio) (100 mg, 0.15 mmol) in CH₂Cl₂ (1 ml) at ambient temperature was added either 1 equivalent of methanesulfonic acid (0.15 mmol, 0.2 ml of 74 mg/ml solution in CH₂Cl₂) or 2 equivalent of methanesulfonic acid (0.3 mmol, 0.4 ml of 74 mg/ml solution in CH₂Cl₂). The resulting homogeneous solution was stirred at ambient temperature for 1 h and was added dropwise to 10 volumes of antisolvents (ethylacetate, methyl tert-butylether (MTBE), or cyclohexane) (10 ml as compared to CH₂Cl₂) resulting in formation of a white solid. The mixture was aged at ambient temperature for 1h and placed at 2-8 °C for 19 h. Upon filtration and washing of the filter cake with the antisolvent and drying, a white solid was obtained. Analysis by XRPD indicated formation of an amorphous solid. Both ¹H-NMR and IC analysis indicated formation of the salt as well as counterion ratio.

Alternatively (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (having about 9/1 E/Z ratio) can be dissolved in 4 volumes of isopropylacetate and added to 2 equivalent of methanesulfonic acid in 6 volumes of isopropylacetate at 0 °C to generate the dimesylate salt.

| Salt form | Solvent | Anti solvent | XRPD | IC-mesylate content¹ |
|---|---|---|---|---|
| 2MSA | CH₂Cl₂ | EtOAc | Amorphous | ND |
| MSA | CH₂Cl₂ | EtOAc | Amorphous | 12.5% |
| 2MSA | CH₂Cl₂ | MTBE | Amorphous | 22.8% |
| MSA | CH₂Cl₂ | MTBE | Amorphous | 14.8% |
| 2MSA | CH₂Cl₂ | Cyclohexane | Amorphous | 21.8% |
| MSA | CH₂Cl₂ | Cyclohexane | Amorphous | 13.9% |
| 2MSA | IPAC | | | ND |

| | | | | |
|---|---|---|---|---|
| 1. Theoretical mesylate content, monomesylate=12.6% and dimesylate=22.4%, ND= not determined | | | | |

### Example 5

### General procedure for the preparation of a carboxylate salt of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile having about 9/1 E/Z ratio

Approximately 20 mg of (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitiile (having about 9/1 E/Z ratio) was dissolved in minimum amount of the allocated solvent system. These were then mixed with the appropriate number of equivalents of counterion dissolved or slurried in the allocated solvent.

If above compound was insoluble in the selected solvent, slurry of the sample was used after adding 300 µL.

If the acid was insoluble in the selected solvent, slurry of the acid was used after adding 300 µL.

If the acid was a liquid, the acid was added to the dissolved/slurried Compound (I) from a stock solution in the allocated solvent.

The suspensions/ precipitates resulting from the mixtures of above compound were temperature cycled between ambient (*ca.* 22°C) and 40°C in 4 hour cycles for *ca.* 48 hrs (the cooling/heating rate after each 4 hour period was *ca.* 1°C/min). The mixtures were visually checked and any solids present were isolated and allowed to dry at ambient conditions prior to analysis. Where no solid was present, samples were allowed to evaporate at ambient. Samples which produced amorphous material, after the treatment outlined above, were redissolved and precipitated using anti-solvent (*tert*-butylmethylether) addition methods at ambient conditions (*ca.* 22°C). *i.e.* the selected anti-solvent was added to each solution, until no further precipitation could be observed visually or until no more anti-solvent could be added. The solvents used in this preparation were acetonitrile, acetone,isopropyl acetate, THF and MTBE. The acidused were oxalic acid, L-aspartic acid, maleic acid, malonic acid, L-tartaric acid, and fumaric acid.

### Example 6

### General procedure for preparation hemicitrate salt of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile having about 9/1 E/Z ratio

To a solution (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (having about 9/1 E/Z ratio) (5 g, 7.5 mmol) in ethanol (50 ml) was added citric acid (720.5 mg, 3.76 mmol) dissolved in 2 ml of water. Mixture was stirred at ambient temperature for 15 min, additional 0.5 ml of water was added and the mixture was stirred for 1 h, concentrated *in vacuo* to a gum. Ethanol was added and the mixture was concentrated. This process was repeated twice more and then CH₂Cl₂ was added to the mixture. Upon concentration a white solid was obtained which was tumble dried under reduced pressure at 40 C for 4 h, then in a vacuum oven for 19h to give 5.4 g of a solid. Analysis by XRD indicated formation of an amorphous solid.

### Example 7

### Osmotically Activated Dosage Form for ileo-jejunal Delivery

The following ingredients are used to prepare an ileo-jejunal dosage form of Compound (I):

| Core layer 1 | |
|---|---|
| | mg |
| Compound (I) | 100 |
| citric acid | 30 |
| lactose | 50 |
| Cabosil® (DSM) | 3.5 |
| Sodium stearoyl fumarate | 3.5 |

| Core layer 2 (osmotic core) | |
|---|---|
| | mg |
| PolyOx™ Coagulant (Dow) | 400 |
| NaCl | 100 |
| Sodium stearoyl fumarate | 3.5 |

| semipermeable membrane | |
|---|---|
| | mg |
| Cellulose acetate NF-398-10 | 2.5 |
| Cellulose acetate NF-320S | 17 |
| Hypomellose USP | 1 |
| Polyethylene glycol 3350 NF | 1 |

| enteric coating | |
|---|---|
| | mg |
| Acyl-EZE® coating solution (Colorcon) | 20% |
| Double distilled water | 100 |

The blend for each core layer is sized and dry blended with the lubricant added last with additional mixing. The 2 blends are tableted by conventional tableting techniques on a rotary bilayer tablet press. A pre-coat of hypromellose solution may optionally also be applied to the uncoated cores. The blends for the tablets are layered in the tablet press and compressed into a tablet with a single compression.

The ingredients for the semipermeable membrane are added to a methylene chloride:methanol (4:1 w/w) solution using a propeller mixer. The uncoated tablets are spray coated and dried.

These coated tablets are then enteric coated with the aqueous solution for different ranges until tablets resist release in acid media for 2 hours, typically with weight gains from 6 to 15% and coating thicknesses ranging from about 50 to about 150 microns.

### Example 8 (comparative example)

### Enteric coated tablet for ileo-jejunal Delivery

200 mg of Compound (I) for each coated tablet is first sized and then dry blended with 7 mg sodium croscarmellose (Ac-Disol®) and 143 mg of microcrystalline cellulose (Avicel® PH 101) in a V-blender for 20 to 30 minutes. Following this 5 mg of sodium stearyl fumarate is added as a lubricant and blended for 4 to 5 minutes. The blend is that tableted on a rotary tablet press using 5/16" standard concave punches. A coating mixture is prepared from 250 g Eudragit® L-30 D-55, 7.5 g triethyl citrate, 37.5 g talc, and 205 g deionized water. The tablet cores are placed in a perforated pan coater rotated at 15 rpm at 40°C. This mixture is sprayed with an inlet air temperature of 44 to 48°C , an exhaust air temperature of 29 to 32°C, a product temperature of 26°C, rotating at 30 to 32 rpm, spray pressure of 20 psi, and an airflow of 30-32 CFM. After curing for 30 minutes with an air inlet temperature of 60°C and rotating at 15 rpm, the heat is turned off, and the tablets cooled to room temperature while rotating. The amount of weight gain after coating is about 5 to about 15%, and typically about 10%. Dissolution times for the enteric coating at pH 6.8 are targeted at greater than 80% in 1 hour.

### Example 9

### Enteric coated granules

Enteric coated granules of a size range from 300 to 500 microns are incorporated for inclusion in capsules, either in gelatin or hypromellose capsules, in sachets or in stickpacks, or in an oral suspension. Compound (I) and low viscosity hypromellose (about 2%) are sized and mixed in a V blender, and then added to a fluid bed granulator. Granules are formed by spraying dilute aqueous polyvinylpyrrolidone solution on to the powder, and then drying the granules in the fluids bed at 45 °C. In the fluid bed, these dried granules are then coated with an Opadry® clear solution in water to provide a seal coat and dried. Eudragit® L-30 D-55 as an aqueous dispersion of 30% polymer, 0.7% sodium lauryl sulfate, and 2.3% Tween® 20 are combined with the plasticizers, triethyl citrate and glyceryl monostearate, and coated on the powder in the fluid bed. After drying the final composition of the enteric-coated granules is about 81.8% active ingredient, about 1.5% hypromellose, about 0.5% Opadry Clear, about 14.5% methacrylic acid copolymer, 1.45% triethyl citrate, and 0.25% glyceryl monostearate. The dried granules are filled into size 0 hypromellose capsules. At pH 2, the capsules dsintegrate and the granules are freed from the capsule, but less than 2% of the drug is in solution at 2 hours.

### Example 10 (comparative example)

### Drug in Enteric Coated Beads

1 kg of the drug and 0.1 kg talc are blended for 15 minutes in a V-blender. Then milled and screened to yield a fine powder. A binder solution is prepared with 10% (w/v) PVP in water. A coating pan is then charged with 1 kg of inert sugar spheres (20 to 50 mesh). The sugar spheres are then sprayed with the binder solution and the drug blend is applied to the spheres until all of the drug was consumed. The drug-loaded beads are then dried in a fluid bed dryer.

A fluidized bed coater is loaded with 1 kg of the above drug-loaded beads. The beads are then coated with 1 kg of the coating solution from Example 7, and then dried. Talc may be used to reduce tackiness during the coating process.

### Example 11

### Ileo-jejunal dosing of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile having about 9/1 E/Z isomer ratio in rats

Compound (I) was dosed in 10 mg/mL aqueous citrate solution at 20 mg/kg to Wister-Hans rats. Free base of Compound (I) was dissolved in aqueous solution of citric acid (containing 0.5 equivalents of anhydrous citiric acid). The experiment was repeated 3 times each with 2 separate arms, and one oral (PO) gavage arm was included in each study. Intra-jejunal (IJ) and intra-duodenal (ID) dosing was done in cannulated rats. In the first study, there was about 40-fold increase in the AUC for the IJ-dosed group compared to the PO group, and the major metabolite, present in the PO arm was reduced by 20 fold in the IJ-group.

### Example 12

### In Vitro Permeation in Ussing Chambers through Different Intestinal Regions of Rat

Reagents: 10X Krebs-Ringer buffer (KRB) comprised of 1.26M NaCl (Promega, Madison, WI), 25mM KCl, 250mM NaHCO₃, 12mM NaH₂PO₄, 12mM MgCh, 25mM CaCl₂ and 18g/L D-Glucose (all from Sigma Aldrich, St. Louis, MO) prepared and stored individually to prevent crystallization. Dilute to IX working concentration prior to use and pH adjusted with either carbogen perfusion for 20 mins to pH 7.4 (oxygenated KRB) or 1M HCl (Alfar Aesar, Ward Hill, MA) to pH 3.5, 6.5 or 7.4. 0.1% Phenol red, Antipyrine and Atenolol were purchased from Sigma Aldrich, St. Louis, MO.

Harvest and preparation of intestinal tissue: 4 to 9 month old adult female virgin Sprague-Drawley rats (Harlan Laboratories, Livermore,CA) were acclimated in-house for a minimum of 3 days prior to experimentation. Adolescent and geriatric animals should be avoided to prevent age-related differences in gastro-intestinal morphology and function. Animals were housed under IACUC-approved husbandry protocols including 12-hour light and 12-hour dark cycles, and allowed standard chow and water *ad libitum.* Tissue harvest should be performed one animal at a time to maintain tissue viability. One at a time, rats were anaesthetized in a sealed gas chamber perfused slowly with 10% (v/v)/min carbon dioxide to minimize distress on the nasal mucosa for approximately 3-5 minutes or until complete sedation as evidenced by lack of response to toe-pinch and shallow breathing. Rats are then quickly euthanized by cervical dislocation.

A modified literature protocol was utilized (D. I. Kosik-Bogacka, et al., (2011) "The effect of L-ascorbic acid and/or tocopherol supplementation on electrophysiological parameters of the colon of rats chronically exposed to lead" Med. Sci .Monit. 2011 17(1):BR16-26). A midline incision was performed to expose the abdominal cavity. The length of the gastro-intestinal tissue from the stomach through the ascending colon was dissected in one piece and placed in a pre-cooled dissection pan filled with ice-cold oxygenated KRB at pH 7.4. Tissues were quickly moved into a fresh dissection pan filled with ice-cold KRB at pH7.4 and continuously bubbled with carbogen gas (95% O₂, 5% CO₂)(CryoSpec, South San Francisco, CA). The different segments of intestine were then separated with a scalpel according to the schematics The stomach comprised *ca.*1.5 inches, the duodenum *ca.* 2 inches, the jejunum *ca.* 8-10 inches, ileum ca. 1 inch and the ascending colon, *ca.* 1 inch.

Each tubular segment was then cut longitudinally along the mesenteric border to expose the luminal surface. Intestinal contents were gently flushed away with care to not disturb the luminal epithelia. Each segment was further cut into smaller pieces measuring approximately 8mm x 10mm, gently stretched and mounted on the pins on one half of a vertical diffusion Ussing chamber (Navicyte®, Harvard Apparatus Inc., Holliston, MA) with an effective surface area of 0.49cm². Mounted segments were visually inspected for tears before fusing with the second half of the chamber. The chambers were immediately filled with 5ml physiological pH matched KRB (Mucosal chambers: Stomach pH 3.5, Duodenum pH 6.5, Jejunum pH 6.5, Ileum pH 7.4, Colon pH 6.5; Serosal chambers: All sections at pH 7.4). Each completed Ussing chamber assembly was serially mounted onto a thermocirculated heat block maintained at 37°C and attached to a gas manifold continuously sparged with carbogen gas at a rate of 3-5 bubbles/second. Phenol red (10 uL 0.1%) was added to each mucosal chamber to check for pH equilibration and evidence of microscopic tears in tissue.

Experimental Protocol: (R)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile having about 9/1 E/Z ratio (experimental samples and control samples) were initially prepared as a 10 mM stock in 100% DMSO. Experimental outline was performed according to (S. Haslam, et al., "Intestinal Ciprofloxacin Efflux: The Role of Breast Cancer Resistance Protein (ABCG2)" Drug Met. Disp. 2011 39(12):2321-28). Briefly, an identical volume of KRB was then removed and replaced by the volume of each compound to obtain a 100 uM starting concentration. Antipyrine and atenolol were included in every experiment as internal reference controls with a maximum of 5 total compounds (including reference controls) in each experiment. Samples (100 uL) were removed from the mucosal chamber at t=0 and t=150min, whilst 100ul samples were removed from the serosal chamber at t=30, 60, 90 and 150 min. The samples were placed in a 96-well plate, frozen at - 80°C for analysis by RapidFire-LC-MS/MS. Apparent permeability, Pₐₚₚ was expressed as Pₐₚₚ= (dQ/dt).(1/(A.C₀)) where dQ/dt is the rate of transport from mucosal to serosal chamber, A is the effective surface area of tissue and C₀ is the initial mucosal concentration (A. Sjoberg, et al., "Comprehensive study on regional human intestinal permeability and prediction of fraction absorbed of drugs using the Ussing chamber technique" Eur. J Pharm. Sci. 2013 48: 166-180).

The results for the above compound are shown in Figure 7. There is generally increased permeability in the distal regions of the GI tract compared to either the stomach or the duodenum, and in particular, the statistically increased permeabilities for the above compound in the jejunal, ileal, and colonic regions.

### Example 13

### Dissolution Testing

Dissolution Testing is carried out in accordance with section 711 of United States Pharmacopeia (http://www.pharmacopeia.cn/v29240/usp29nf24s0_c711h.html) using apparatus 2 (paddle at 75 rpm) with sinker alternative 2A. Baths containing 900 mL for both acid (pH 2 or 3) and pH 6.8 tests at 37 °C (± 0.5 °C). To study delivery to the ileum, dissolution at pH 7.4 may also be performed. If dissolution testing is performed in the range above pH 6, additives (such as surfactanats or cyclodextrins) must be added to the media to dissolve Compound (I) and ensure sink conditions.

## Claims

1. A solid oral dosage form, which is typically a tablet or capsule, comprising:
(A) a core material comprising the (E) isomer, (Z) isomer, or a mixture of (E) and (Z) isomers of (R)-2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile, (S)-2-[3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile, or a mixture of (R) and (S) isomers of 2-[3-[4-amino-3-(2-fluoro-4-phenoxy-phenyl)pyrazolo[3,4-d]pyrimidin-1-yl]piperidine-1-carbonyl]-4-methyl-4-[4-(oxetan-3-yl)piperazin-1-yl]pent-2-enenitrile (Compound (I)) having the structure: 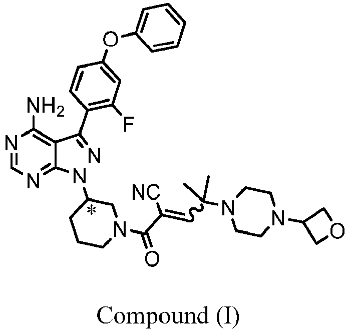
where *C is a stereochemical center;
and/or a pharmaceutically acceptable salt thereof;
(B) an enteric coating;
(C) a subcoat below the enteric coating, wherein the subcoat:
(1) is a water soluble or hydrophilic erodible polymer, said polymer being a low molecular weight polymer selected from hydroxymethyl cellulose (HPMC), hydroxyethyl cellulose, hydroxypropyl cellulose, microcrystalline cellulose, polyvinylpyrrolidones, polysaccharides, a polysaccharide derivative, polyvinyl alcohols, polyethylene glycol (PEG), polypropylene glycol (PPG), and a PEG-PPG block copolymer; or
(2) comprises a water insoluble composition comprising: (i) particles of a water soluble compound capable of forming channels in the water insoluble composition; or (ii) water insoluble hydrophilic particles which cause swelling of the subcoat when in contact with an aqueous media; and
(D) a pharmaceutically acceptable excipient,
wherein the solid oral dosage form has an onset of release of Compound (I) and/or the pharmaceutically acceptable salt thereof in the jejuno-ileum portion of the intestine of a mammal after administration; and
wherein the solid oral dosage form releases at least 80% of Compound (I) and/or the pharmaceutically acceptable salt thereof in 120 minutes in a dissolution vessel comprising an aqueous solution at a pH from 6.4 to 7.4.

2. The solid oral dosage form according to claim 1, wherein the solid oral dosage form releases less than 10% by weight of Compound (I) and/or the pharmaceutically acceptable salt thereof in 1.5 hours in a dissolution vessel comprising an aqueous solution at a pH of less than 3 or a pH from 4.5 to 5.0; and wherein the solid oral dosage form releases not less than 80% by weight of Compound (I) and/or the pharmaceutically acceptable salt thereof from twenty minutes to two hours in a dissolution vessel comprising an aqueous solution at a pH from 6.4 to 7.4.

3. The solid oral dosage form according to claim 1 or claim 2, wherein the subcoat (C):
(a) comprises a water insoluble composition comprising particles of a water soluble compound;
(b) comprises a water insoluble composition comprising water insoluble hydrophilic particles which cause swelling of the subcoat when in contact with an aqueous media;
(c) is a water insoluble composition comprising particles of a water soluble compound capable of forming channels in the water insoluble composition causing influx of water into the solid oral dosage form and diffusion of Compound (I) and/or the pharmaceutically acceptable salt thereof into the intestine; or
(d) is a water insoluble composition comprising particles of a water soluble compound capable of forming channels that are impermeable to Compound (I) and/or the pharmaceutically acceptable salt thereof, but which allow entry of water and swelling and rupturing of the subcoat, causing release of Compound (I) and/or the pharmaceutically acceptable salt thereof.

4. The solid oral dosage form according to any one of claims 1 to 3, wherein the aqueous solution is a simulated intestinal fluid at a pH from 6.4 to 7.4.

5. The solid oral dosage form according to any one of claims 1 to 4, wherein the solid oral dosage form comprises a pharmaceutically acceptable acid salt of Compound (I).

6. The solid oral dosage form according to any one of claims 1 to 5, wherein the core material (A) further comprises:
(a) a pharmaceutically acceptable acid in an amount sufficient to form an acidic aqueous solution within the solid oral dosage form prior to the release of Compound (I) and/or the pharmaceutically acceptable salt thereof from the solid oral dosage form; and/or
(b) a surfactant which is present at a concentration above its critical micelle concentration upon disintegration in 50 mL of aqueous media.

7. The solid oral dosage form according to any one of claims 1 to 6, wherein the mean particle size of Compound (I) and/or the pharmaceutically acceptable salt thereof is from 0.3 micron to 100 microns.

8. The solid oral dosage form according to any one of claims 1 to 7, wherein the enteric coating (B):
(a) is from 5 to 500 microns thick; and/or
(b) is selected from polymerized gelatin, shellac, methacrylic acid copolymer type CNF, cellulose butyrate phthalate, cellulose hydrogen phthalate, cellulose proprionate phthalate, polyvinyl acetate phthalate (PVAP), cellulose acetate phthalate (CAP), cellulose acetate trimellitate (CAT), hydroxypropyl methylcellulose phthalate, hydroxypropyl methylcellulose acetate, dioxypropyl methylcellulose succinate, carboxymethyl ethylcellulose (CMEC), hydroxypropyl methylcellulose acetate succinate (HPMCAS), and (meth)acrylic acid polymers and copolymers, wherein said polymers are made from one monomer and said copolymers are made from two or more monomers, wherein said monomers are selected from methyl acrylate, ethyl acrylate, methyl methacrylate and ethyl methacrylate, preferably wherein the enteric coating comprises a poly(meth)acrylate polymer; and/or
(c) comprises a cellulose derivative, preferably wherein the cellulose derivative is selected from methylcellulose, cellulose acetate phthalate, hydroxymethyl cellulose phthalate (HPMCP), hydroxypropyl methyl cellulose succinate (HPMCS), and hydroxymethyl cellulose acetate succinate (HPMCAS); and/or
(d) comprises a polyvinyl acetate phthalate (PVAP) polymer.

9. The solid oral dosage form according to any one of claims 1 to 8, wherein the pharmaceutically acceptable excipient (D) is independently selected from binders, surfactants, diluents, buffers, antiadherents, glidants, disintegrants, antioxidants, antifoaming agents, fillers, flavors, colors, lubricants, sorbents, preservatives, plasticizers, and sweeteners.

10. The solid oral dosage form according to any one of claims 1 to 9, wherein Compound (I) is an (E) and (Z) mixture of a mixture of (R) and (S) isomers of 2-[3-[4-amino-3-(2-fluoro-4-phenoxy-phenyl)-pyrazolo[3,4-d]pyrimidin-1-yl]piperidine-1-carbonyl]-4-methyl-4-[4-(oxetan-3-yl)piperazin-1-yl]pent-2-enenitrile.

11. The solid oral dosage form according to any one of claims 1 to 9, wherein Compound (I) is an (E) and (Z) mixture of (R)-2-[3-[4-amino-3-(2-fluoro-4-phenoxy-phenyl)-pyrazolo[3,4-d]pyrimidin-1-yl]piperidine-1-carbonyl]-4-methyl-4-[4-(oxetan-3-yl)piperazin-1-yl]pent-2-enenitrile.

12. The solid oral dosage form according to any one of claims 1 to 11, wherein at least 85% by weight of Compound (I) and/or the pharmaceutically acceptable salt thereof is the (E) isomer.

13. The solid oral dosage form according to any one of claims 1 to 12, wherein Compound (I) and/or the pharmaceutically acceptable salt thereof is a substantially pure amorphous form.

14. The solid oral dosage form according to any one of claims 1 to 13 for use in a method of treating a disease treatable by the inhibition of BTK in a patient in recognized need thereof, wherein said method comprises administering to the patient a therapeutically effective amount of Compound (I) and/or the pharmaceutically acceptable salt thereof.

15. The solid oral dosage form for use according to claim 14, wherein the disease is selected from an autoimmune disease, cancer, and an inflammatory disease, preferably a leukemia or lymphoma, and further optionally wherein the leukemia or lymphoma is selected from chronic lymphocytic leukemia (CLL), small lymphocytic lymphoma (SLL), multiple myeloma, mantle cell lymphoma, and B-cell non-Hodgkin lymphoma.

## Patentansprüche

1. Feste orale Dosierungsform, die typischerweise eine Tablette oder Kapsel ist, umfassend:
(A) ein Kernmaterial, umfassend das (E)-Isomer, (Z)-Isomer oder ein Gemisch aus (E)- und (Z)-Isomeren von (R)-2-[3-[4-Amino-3-(2-fluoro-4-phenoxyphenyl)pyrazolo[3,4-d]pyrimidin-1-yl]piperidin-1-carbonyl]-4-methyl-4-[4-(oxetan-3-yl)piperazin-1-yl]pent-2-enenitril, (S)-2-[3-[4-Amino-3-(2-fluoro-4-phenoxyphenyl)pyrazolo[3,4-d]pyrimidin-1-yl]piperidin-1-carbonyl]-4-methyl-4-[4-(oxetan-3-yl)piperazin-1-yl]pent-2-enenitril oder ein Gemisch der (R)- und (S)-Isomere von 2-[3-[4-Amino-3-(2-fluor-4-phenoxyphenyl)pyrazolo[3,4-d]pyrimidin-1-yl]piperidin-1-carbonyl]-4-methyl-4-[4-(oxetan-3-yl)piperazin-1-yl]pent-2-enenitril (Verbindung (I)) mit der Struktur: 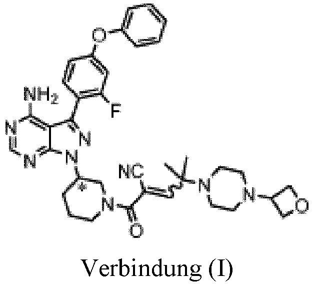
wobei *C ein stereochemisches Zentrum ist;
und/oder ein pharmazeutisch unbedenkliches Salz davon;
(B) eine magensaftresistente Beschichtung;
(C) eine Unterschicht unter der magensaftresistenten Beschichtung, wobei die Unterschicht:
(1) ein wasserlösliches oder hydrophiles erodierbares Polymer ist, wobei das Polymer ein Polymer mit niedrigem Molekulargewicht ist, ausgewählt aus Hydroxymethylcellulose (HPMC), Hydroxyethylcellulose, Hydroxypropylcellulose, mikrokristalliner Cellulose, Polyvinylpyrrolidonen, Polysacchariden, einem Polysaccharidderivat, Polyvinylalkoholen, Polyethylenglykol (PEG), Polypropylenglykol (PPG) und einem PEG-PPG-Blockcopolymer; oder
(2) eine wasserunlösliche Zusammensetzung umfasst, die umfasst: (i) Teilchen einer wasserlöslichen Verbindung, die in der Lage sind, Kanäle in der wasserunlöslichen Zusammensetzung auszubilden; oder (ii) wasserunlösliche hydrophile Teilchen, die bei Kontakt mit einem wässrigen Medium ein Aufquellen der Unterschicht verursachen; und
(D) einen pharmazeutisch unbedenklichen Hilfsstoff,
wobei die feste orale Dosierungsform einen Beginn der Freisetzung von Verbindung (I) und/oder des pharmazeutisch unbedenklichen Salzes davon in dem Jejuno-Ileum-Abschnitt des Darms eines Säugetiers nach Verabreichung aufweist; und
wobei die feste orale Dosierungsform wenigstens 80 % von Verbindung (I) und/oder dem pharmazeutisch unbedenklichen Salz davon in 120 Minuten in einem Auflösungsgefäß, das eine wässrige Lösung bei einem pH-Wert von 6,4 bis 7,4 umfasst, freisetzt

2. Feste orale Dosierungsform nach Anspruch 1, wobei die feste orale Dosierungsform weniger als 10 Gew.-% der Verbindung (I) und/oder des pharmazeutisch unbedenklichen Salzes davon in 1,5 Stunden in einem Auflösungsgefäß, das eine wässrige Lösung bei einem pH-Wert von weniger als 3 oder einem pH-Wert von 4,5 bis 5,0 umfasst, freisetzt; und wobei die feste orale Dosierungsform nicht weniger als 80 Gew.-% der Verbindung (I) und/oder des pharmazeutisch unbedenklichen Salzes davon in zwanzig Minuten bis zwei Stunden in einem Auflösungsgefäß, das eine wässrige Lösung bei einem pH-Wert von 6,4 bis 7,4 umfasst, freisetzt.

3. Feste orale Dosierungsform nach Anspruch 1 oder 2, wobei die Unterschicht (C):
(a) eine wasserunlösliche Zusammensetzung umfasst, die Teilchen einer wasserlöslichen Verbindung umfasst;
(b) eine wasserunlösliche Zusammensetzung umfasst, die wasserunlösliche hydrophile Teilchen umfasst, die ein Aufquellen der Unterschicht verursachen, wenn sie mit einem wässrigen Medium in Kontakt kommen;
(c) eine wasserunlösliche Zusammensetzung ist, die Teilchen einer wasserlöslichen Verbindung umfasst, die in der Lage sind, Kanäle in der wasserunlöslichen Zusammensetzung auszubilden, die ein Einströmen von Wasser in die feste orale Dosierungsform und die Diffusion von Verbindung (I) und/oder des pharmazeutisch unbedenklichen Salzes davon in den Darm verursachen; oder
(d) eine wasserunlösliche Zusammensetzung ist, die Teilchen einer wasserlöslichen Verbindung umfasst, die in der Lage sind, Kanäle auszubilden, die für Verbindung (I) und/oder das pharmazeutisch unbedenkliche Salz davon undurchlässig sind, die jedoch den Eintritt von Wasser und das Aufquellen und Aufbrechen der Unterschicht ermöglichen, was die Freisetzung von Verbindung (I) und/oder des pharmazeutisch unbedenklichen Salzes davon verursacht.

4. Feste orale Dosierungsform nach einem der Ansprüche 1 bis 3, wobei die wässrige Lösung eine simulierte Darmflüssigkeit mit einem pH-Wert von 6,4 bis 7,4 ist.

5. Feste orale Dosierungsform nach einem der Ansprüche 1 bis 4, wobei die feste orale Dosierungsform ein pharmazeutisch unbedenkliches Säuresalz der Verbindung (I) umfasst.

6. Feste orale Dosierungsform nach einem der Ansprüche 1 bis 5, wobei das Kernmaterial (A) ferner umfasst:
(a) eine pharmazeutisch unbedenkliche Säure in einer Menge, die ausreicht, um eine saure wässrige Lösung innerhalb der festen oralen Dosierungsform vor der Freisetzung von Verbindung (I) und/oder des pharmazeutisch unbedenklichen Salzes davon aus der festen oralen Dosierungsform auszubilden; und/oder
(b) ein Tensid, das in einer Konzentration oberhalb seiner kritischen Mizellenkonzentration beim Zerfall in 50 ml wässrigem Medium vorhanden ist.

7. Feste orale Dosierungsform nach einem der Ansprüche 1 bis 6, wobei die mittlere Teilchengröße der Verbindung (I) und/oder des pharmazeutisch unbedenklichen Salzes davon 0,3 Mikron bis 100 Mikron beträgt.

8. Feste orale Dosierungsform nach einem der Ansprüche 1 bis 7, wobei die magensaftresistente Beschichtung (B):
(a) eine Dicke von 5 bis 500 Mikron aufweist; und/oder
(b) aus polymerisierter Gelatine, Schellack, Methacrylsäure-Copolymer Typ CNF, Cellulosebutyratphthalat, Cellulosehydrogenphthalat, Celluloseproprionatphthalat, Polyvinylacetatphthalat (PVAP), Celluloseacetatphthalat (CAP), Celluloseacetattrimellitat (CAT), Hydroxypropylmethylcellulosephthalat, Hydroxypropylmethylcelluloseacetat, Dioxypropylmethylcellulosesuccinat, Carboxymethylethylcellulose (CMEC), Hydroxypropylmethylcelluloseacetatsuccinat (HPMCAS) und (Meth)acrylsäure-Polymere und -Copolymere ausgewählt ist, wobei diese Polymere aus einem Monomer hergestellt sind und die Copolymere aus zwei oder mehr Monomeren hergestellt sind, wobei die Monomere aus Methylacrylat, Ethylacrylat, Methylmethacrylat und Ethylmethacrylat ausgewählt sind, wobei vorzugsweise die magensaftresistente Beschichtung ein Poly(meth)acrylat-Polymer umfasst; und/oder
(c) ein Cellulosederivat umfasst, wobei das Cellulosederivat vorzugsweise aus Methylcellulose, Celluloseacetatphthalat, Hydroxymethylcellulosephthalat (HPMCP), Hydroxypropylmethylcellulosesuccinat (HPMCS) und Hydroxymethylcelluloseacetatsuccinat (HPMCAS) ausgewählt ist; und/oder
(d) ein Polyvinylacetatphthalat (PVAP)-Polymer umfasst.

9. Feste orale Dosierungsform nach einem der Ansprüche 1 bis 8, wobei der pharmazeutisch unbedenkliche Hilfsstoff (D) unabhängig aus Bindemitteln, Tensiden, Verdünnungsmitteln, Puffern, Antihaftmitteln, Gleitmitteln, Sprengmitteln, Antioxidantien, Antischaummitteln, Füllstoffen, Geschmacksstoffen, Farben, Schmiermitteln, Sorptionsmitteln, Konservierungsmitteln, Weichmachern und Süßstoffen ausgewählt ist.

10. Feste orale Dosierungsform nach einem der Ansprüche 1 bis 9, wobei Verbindung (I) ein (E) und (Z) Gemisch aus einem Gemisch von (R)- und (S)-Isomeren von 2-[3-[4-Amino-3-(2-fluoro-4-phenoxy-phenyl)-pyrazolo[3,4-d]pyrimidin-1-yl]piperidin-1-carbonyl]-4-methyl-4-[4-(oxetan-3-yl)piperazin-1-yl]pent-2-enenitril ist.

11. Feste orale Dosierungsform nach einem der Ansprüche 1 bis 9, wobei die Verbindung (I) ein (E) und (Z) Gemisch aus (R)-2-[3-[4-Amino-3-(2-fluoro-4-phenoxyphenyl)pyrazolo[3,4-d]pyrimidin-1-yl ]piperidin-1-carbonyl]-4-methyl-4-[4-(oxetan-3-yl)piperazin-1-yl]pent-2-enenitril ist.

12. Feste orale Dosierungsform nach einem der Ansprüche 1 bis 11, wobei wenigstens 85 Gew.-% der Verbindung (I) und/oder des pharmazeutisch unbedenklichen Salzes davon das (E)-Isomer ist.

13. Feste orale Dosierungsform nach einem der Ansprüche 1 bis 12, wobei die Verbindung (I) und/oder das pharmazeutisch unbedenkliche Salz davon eine im Wesentlichen reine amorphe Form ist.

14. Feste orale Dosierungsform nach einem der Ansprüche 1 bis 13 für die Verwendung in einem Verfahren zum Behandeln einer Krankheit, die durch die Hemmung von BTK behandelbar ist, bei einem Patienten mit erkanntem Bedarf daran, wobei das Verfahren das Verabreichen einer therapeutisch wirksamen Menge der Verbindung (I) und/oder des pharmazeutisch unbedenklichen Salzes davon an den Patienten umfasst.

15. Feste orale Dosierungsform für die Verwendung nach Anspruch 14, wobei die Krankheit aus einer Autoimmunkrankheit, Krebs und einer Entzündungskrankheit, vorzugsweise einer Leukämie oder einem Lymphom, ausgewählt ist und wobei ferner optional die Leukämie oder das Lymphom aus chronischer lymphatischer Leukämie (CLL), kleinem lymphatischem Lymphom (SLL), multiplem Myelom, Mantelzell-Lymphom und B-Zell-Non-Hodgkin-Lymphom ausgewählt ist.

## Revendications

1. Forme de dosage orale solide, qui est typiquement un comprimé ou une capsule, comprenant :
(A) un matériau central comprenant l'isomère (E), l'isomère (Z), ou un mélange d'isomères (E) et (Z) de (R)-2-[3-[4-amino-3-(2-fluoro-4-phénoxy-phényl)pyrazolo[3,4-d]pyrimidin-1-yl]pipéridine-1-carbonyl]-4-méthyl-4-[4-(oxétan-3-yl)pipérazin-1-yl]pent-2-ènenitrile, de (S)-2-[3-[4-amino-3-(2-fluoro-4-phénoxy-phényl)pyrazolo[3,4-d]pyrimidin-1-yl]pipéridine-1-carbonyl]-4-méthyl-4-[4-(oxétan-3-yl)pipérazin-1-yl]pent-2-ènenitrile, ou un mélange d'isomères (R) et (S) de 2-[3-[4-amino-3-(2-fluoro-4-phénoxy-phényl)pyrazolo[3,4-d]pyrimidin-1-yl]pipéridine-1-carbonyl]-4-méthyl-4-[4-(oxétan-3-yl)pipérazin-1-yl]pent-2-ènenitrile (composé (I)) ayant la structure : 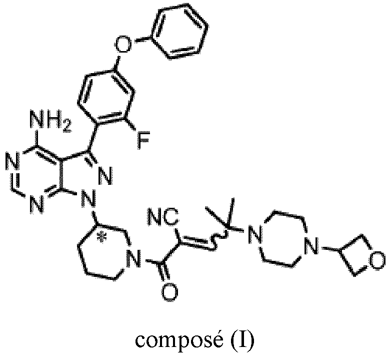
où *C est un centre stéréochimique ;
et/ou un sel pharmaceutiquement acceptable de celui-ci ;
(B) un enrobage entérique ;
(C) une sous-couche en-dessous de l'enrobage entérique, dans lequel la sous-couche :
(1) est un polymère érodable hydrosoluble ou hydrophile, ledit polymère étant un polymère de faible poids moléculaire sélectionné parmi : hydroxyméthylcellulose (HPMC), hydroxyéthylcellulose, hydroxypropylcellulose, cellulose microcristalline, polyvinylpyrrolidones, polysaccharides, un dérivé de polysaccharide, alcools de polyvinyle, polyéthylèneglycol (PEG), polypropylèneglycol (PPG), et un copolymère bloc PEG-PPG ; ou
(2) comprend une composition hydro-insoluble comprenant : (i) des particules d'un composé hydrosoluble capable de former des canaux dans la composition hydro-insoluble ; ou (ii) des particules hydrophiles hydro-insolubles qui causent le gonflement de la sous-couche lors du contact avec un milieu aqueux ; et
(D) un excipient pharmaceutiquement acceptable,
dans lequel la forme de dosage orale solide a un début de libération du composé (I) et/ou du sel pharmaceutiquement acceptable de celui-ci dans la partie jéjuno-iléon de l'intestin d'un mammifère après l'administration ; et
dans lequel la forme de dosage orale solide libère au moins 80 % du composé (I) et/ou du sel pharmaceutiquement acceptable de celui-ci en 120 minutes dans un récipient de dissolution comprenant une solution aqueuse à un pH de 6,4 à 7,4.

2. Forme de dosage orale solide selon la revendication 1, dans lequel la forme de dosage orale solide libère moins de 10 % en poids du composé (I) et/ou du sel pharmaceutiquement acceptable de celui-ci en 1,5 heures dans un récipient de dissolution comprenant une solution aqueuse à un pH inférieur à 3 ou un pH de 4,5 à 5,0 ; et dans lequel la forme de dosage orale solide ne libère pas moins de 80 % en poids du composé (I) et/ou du sel pharmaceutiquement acceptable de celui-ci entre vingt minutes et deux heures dans un récipient de dissolution comprenant une solution aqueuse à un pH de 6,4 à 7,4.

3. Forme de dosage orale solide selon la revendication 1 ou la revendication 2, dans lequel la sous-couche (C) :
(a) comprend une composition hydro-insoluble comprenant des particules d'un composé hydrosoluble ;
(b) comprend une composition hydro-insoluble comprenant des particules hydrophiles hydro-insolubles qui causent le gonflement de la sous-couche lors du contact avec un milieu aqueux ;
(c) est une composition hydro-insoluble comprenant des particules d'un composé hydrosoluble capable de former des canaux dans la composition hydro-insoluble causant l'afflux d'eau dans la forme de dosage orale solide et la diffusion du composé (I) et/ou du sel pharmaceutiquement acceptable de celui-ci dans l'intestin ; ou
(d) est une composition hydro-insoluble comprenant des particules d'un composé hydrosoluble capable de former des canaux qui sont imperméables au composé (I) et/ou au sel pharmaceutiquement acceptable de celui-ci, mais qui permettent l'entrée d'eau et le gonflement et la rupture de la sous-couche, causant la libération du composé (I) et/ou du sel pharmaceutiquement acceptable de celui-ci.

4. Forme de dosage orale solide selon l'une quelconque des revendications 1 à 3, dans lequel la solution aqueuse est un fluide intestinal simulé à un pH de 6,4 à 7,4.

5. Forme de dosage orale solide selon l'une quelconque des revendications 1 à 4, dans lequel la forme de dosage orale solide comprend un sel d'acide pharmaceutiquement acceptable du composé (I).

6. Forme de dosage orale solide selon l'une quelconque des revendications 1 à 5, dans lequel le matériau central (A) comprend en outre :
(a) un acide pharmaceutiquement acceptable en une quantité suffisante pour former une solution aqueuse acide à l'intérieur de la forme de dosage orale solide avant la libération du composé (I) et/ou du sel pharmaceutiquement acceptable de celui-ci à partir de la forme de dosage orale solide ; et/ou
(b) un surfactant qui est présent à une concentration supérieure à sa concentration micellaire critique lors de la désintégration dans 50 mL de milieu aqueux.

7. Forme de dosage orale solide selon l'une quelconque des revendications 1 à 6, dans lequel la taille moyenne des particules du composé (I) et/ou du sel pharmaceutiquement acceptable de celui-ci est de 0,3 micron à 100 microns.

8. Forme de dosage orale solide selon l'une quelconque des revendications 1 à 7, dans lequel l'enrobage entérique (B) :
(a) fait de 5 à 500 microns d'épaisseur ; et/ou
(b) est sélectionné parmi : gélatine polymérisée, gomme laque, copolymère d'acide méthacrylique type CNF, butyrate phtalate de cellulose, phtalate hydrogéné de cellulose, proprionate phtalate de cellulose, acétate phtalate de polyvinyle (PVAP), acétate phtalate de cellulose (CAP), trimellitate acétate de cellulose (CAT), phtalate d'hydroxypropylméthylcellulose, acétate d'hydroxypropylméthylcellulose, succinate de dioxypropylméthylcellulose, carboxyméthyléthylcellulose (CMEC), acétate succinate d'hydroxypropylméthylcellulose (HPMCAS), et polymères et copolymères d'acide (méth)acrylique, dans lequel lesdits polymères sont faits à partir d'un monomère et lesdits copolymères sont faits à partir de deux, ou plus, monomères, dans lequel lesdits monomères sont sélectionnés parmi : acrylate de méthyle, acrylate d'éthyle, méthacrylate de méthyle et méthacrylate d'éthyle, de préférence dans lequel l'enrobage entérique comprend un polymère de poly(méth)acrylate ; et/ou
(c) comprend un dérivé de cellulose, de préférence dans lequel le dérivé de cellulose est sélectionné parmi : méthylcellulose, acétate phtalate de cellulose, phtalate d'hydroxyméthylcellulose (HPMCP), succinate d'hydroxypropylméthylcellulose (HPMCS), et acétate succinate d'hydroxyméthylcellulose (HPMCAS) ; et/ou
(d) comprend un polymère acétate phtalate de polyvinyle (PVAP).

9. Forme de dosage orale solide selon l'une quelconque des revendications 1 à 8, dans lequel l'excipient pharmaceutiquement acceptable (D) est indépendamment sélectionné parmi : des liants, surfactants, diluants, tampons, anti-adhérents, glissants, désintégrants, antioxydants, agents anti-mousse, charges, arômes, couleurs, lubrifiants, sorbants, préservateurs, plastifiants, et édulcorants.

10. Forme de dosage orale solide selon l'une quelconque des revendications 1 à 9, dans lequel composé (I) est un mélange (E) et (Z) d'un mélange d'isomères (R) et (S) de 2-[3-[4-amino-3-(2-fluoro-4-phénoxy-phényl)-pyrazolo[3,4-d]pyrimidin-1-yl]pipéridine-1-carbonyl]-4-méthyl-4-[4-(oxétan-3-yl)pipérazin-1-yl]pent-2-ènenitrile.

11. Forme de dosage orale solide selon l'une quelconque des revendications 1 à 9, dans lequel composé (I) est un mélange (E) et (Z) de (R)-2-[3-[4-amino-3-(2-fluoro-4-phénoxy-phényl)pyrazolo[3,4-d]pyiimidin-1-yl]pipéridine-1-carbonyl]-4-méthyl-4-[4-(oxétan-3-yl)pipérazin-1-yl]pent-2-ènenitrile.

12. Forme de dosage orale solide selon l'une quelconque des revendications 1 à 11, dans lequel au moins 85 % en poids du composé (I) et/ou du sel pharmaceutiquement acceptable de celui-ci est l'isomère (E).

13. Forme de dosage orale solide selon l'une quelconque des revendications 1 à 12, dans lequel le composé (I) et/ou le sel pharmaceutiquement acceptable de celui-ci est une forme amorphe sensiblement pure.

14. Forme de dosage orale solide selon l'une quelconque des revendications 1 à 13 pour l'utilisation dans un procédé de traitement d'une maladie traitable par l'inhibition de BTK chez un patient en ayant un besoin reconnu, dans lequel ledit procédé comprend l'administration, au patient, d'une quantité thérapeutiquement efficace du composé (I) et/ou du sel pharmaceutiquement acceptable de celui-ci.

15. Forme de dosage orale solide pour l'utilisation selon la revendication 14, dans lequel la maladie est sélectionnée parmi : une maladie auto-immunitaire, un cancer, et une maladie inflammatoire, de préférence une leucémie ou un lymphome, et en outre optionnellement dans lequel la leucémie ou le lymphome est sélectionné parmi : leucémie lymphocytaire chronique (CLL), lymphome à petits lymphocytes (SLL), myélome multiple, lymphome à cellules du manteau, et lymphome non hodgkinien des cellules B.
